(19) Europäisches Patentamt
European Patent Office
Office européen des brevets

(11) **EP 3 638 103 B1**

(12) **EUROPÄISCHE PATENTSCHRIFT**

(45) Veröffentlichungstag und Bekanntmachung des Hinweises auf die Patenterteilung:
**03.09.2025   Patentblatt 2025/36**

(21) Anmeldenummer: **18734139.1**

(22) Anmeldetag: **15.06.2018**

(51) Internationale Patentklassifikation (IPC):
**A61B 5/00** (2006.01)    **A61B 5/12** (2006.01)
**H04R 1/26** (2006.01)    **H04R 25/00** (2006.01)

(52) Gemeinsame Patentklassifikation (CPC):
**A61B 5/125; A61B 5/6817; A61B 5/7235;
A61B 5/7271; H04R 25/70;** A61B 5/7203;
H04R 1/26

(86) Internationale Anmeldenummer:
**PCT/EP2018/066033**

(87) Internationale Veröffentlichungsnummer:
**WO 2018/229287 (20.12.2018 Gazette 2018/51)**

(54) **VERFAHREN UND VORRICHTUNG ZUR UNTERSUCHUNG DES HÖRVERMÖGENS**

METHOD AND DEVICE FOR EXAMINING THE ABILITY TO HEAR

PROCÉDÉ ET DISPOSITIF POUR ÉTUDIER L'AUDITION

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **16.06.2017   DE 102017005675**

(43) Veröffentlichungstag der Anmeldung:
**22.04.2020   Patentblatt 2020/17**

(73) Patentinhaber: **Eberhard-Karls-Universität
Tübingen
72074 Tübingen (DE)**

(72) Erfinder:
• **DALHOFF, Ernst
72108 Rottenburg (DE)**
• **ZELLE, Dennis
72072 Tübingen (DE)**

(74) Vertreter: **Graf von Stosch
Patentanwaltsgesellschaft mbH
Triftstraße 5
80538 München (DE)**

(56) Entgegenhaltungen:
**WO-A1-2015/192969**

• **M. L. WHITEHEAD ET AL: "Dependence of distortion-product otoacoustic emissions on primary levels in normal and impaired ears. II. Asymmetry in L 1 , L 2 space", THE JOURNAL OF THE ACOUSTICAL SOCIETY OF AMERICA, vol. 97, no. 4, 1 April 1995 (1995-04-01), New York, NY, US, pages 2359 - 2377, XP055378625, ISSN: 0001-4966, DOI: 10.1121/1.411960**
• **ZELLE DENNIS ET AL: "Level dependence of the nonlinear-distortion component of distortion-product otoacoustic emissions in humans", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 138, no. 6, 8 December 2015 (2015-12-08), pages 3475 - 3490, XP012203260, ISSN: 0001-4966, [retrieved on 19010101], DOI: 10.1121/1.4936860**
• **ZELLE D ET AL: "Objektive Hördiagnostik mit DPAOE: Neue Erkenntnisse zur Generierung und klinischen Anwendung", HNO. HALS-, NASEN-, OHRENAERZTE, SPRINGER VERLAG, BERLIN, DE, vol. 64, no. 11, 19 October 2016 (2016-10-19), pages 822 - 830, XP036090955, ISSN: 0017-6192, [retrieved on 20161019], DOI: 10.1007/ S00106-016-0254-3**

**EP 3 638 103 B1**

**Beschreibung**

Die Erfindung betrifft ein Verfahren, das auf der Messung von Distorsionsprodukten otoakustischer Emissionen (DPOAE) basiert. Das erfindungsgemäße Verfahren dient insbesondere zur objektiven und quantitativen Ermittlung der Schallverarbeitung in einem Säugerohr und somit der Untersuchung und nachfolgenden Bewertung des Hörvermögens. Die Messergebnisse können gemäß der EP 2 053 877 A1 und der DE 199 05 743 A1 auch zur Einstellung von Hörgeräten verwendet werden. Das Verfahren beruht auf einem speziellen Anregungsmuster, welches Rückschlüsse auf den Arbeitspunkt des cochleären Verstärkers erlaubt.

Insbesondere betrifft die vorliegende Erfindung ein Verfahren zur Erfassung von Distorsionsprodukten otoakustischer Emissionen (DPOAE) in einem Hörorgan, umfassend die Schritte: (a) Ausgabe mindestens eines ersten Primärtonpaares aus je einem ersten Primärton mit Frequenz $f_{1,1}$ und Schalldruckpegel $L_{1,1}$ und einem zweiten Primärton mit Frequenz $f_{2,1}$ und Schalldruckpegel $L_{2,1}$ mit $f_{2,1} > f_{1,1}$, und (b) Erfassen evozierter Distorsionsprodukte otoakustischer Emissionen (DPOAE), dadurch gekennzeichnet, dass der erste Primärton $\{f_{1,1}, L_{1,1}\}$ mit einer zeitlichen Verzögerung $t_{\text{lag}}$ nach dem zweiten Primärton $\{f_{2,1}, L_{2,1}\}$ ausgegeben wird.

Das Hörsystem kann als eine Kette von aufeinanderfolgenden Signalverarbeitungsblöcken angesehen werden. Diese werden durchlaufen, bevor im Cortex die komplexere Wahrnehmung des Hörens entsteht. Die ersten Blöcke der Signalverarbeitungskette sind das äußere Ohr (Ohrmuschel & Gehörgang), das Mittelohr (Gehörknöchelchen mit der Fußplatte als Grenze zu den Flüssigkeiten des Innenohrs), und das flüssigkeitsgefüllte Innenohr. Die allermeisten Hörschäden entstehen im Innenohr. Darunter fällt auch die Altersschwerhörigkeit, die im Mittel im Alter von 60-70 Jahren zu 25 dB Hörverlust bei Frauen bzw. 35 dB bei Männern im Frequenzbereich ab 4 kHz führt. Sie ist dominiert durch eine Beeinträchtigung des sog. cochleären Verstärkers, der im gesunden Zustand die eingehenden Schallwellen um den Faktor 300-1000 verstärkt, bevor sie von den inneren Haarzellen und deren Synapsen in neurale Signale umgewandelt werden.

Seit etwa 1980 ist die Existenz des cochleären Verstärkers sukzessive nachgewiesen worden, und dabei spielte die Entdeckung der otoakustischen Emissionen (OAE) durch David T. Kemp eine zentrale Rolle. Diese sind Töne, die vom aktiven Verstärker als Nebenprodukt erzeugt und durch das Mittelohr rückwärts zum Gehörgang übertragen werden. Dort können sie mit empfindlichen Miniaturmikrophonen gemessen werden.

Eine Form der OAE sind die Distorsionsprodukt-otoakustischen Emissionen (DPOAE), die üblicherweise infolge der simultanen Präsentation zweier Primärtöne mit den Frequenzen $f_1$ und $f_2$ und den (Schalldruck-)Pegeln $L_1$ und $L_2$ entstehen. Die nichtlineare Kennlinie der mechanoelektrischen Transduktion der Ionenkanäle der äußeren Haarzellen, die das hauptsächliche Motorelement des cochleären Verstärkers beim Menschen und den Säugetieren allgemein darstellen, führt zu zahlreichen Distorsionsprodukten. Das am einfachsten messbare Distorsionsprodukt und daher in diagnostischen Anwendungen bevorzugte ist dasjenige bei $f_{dp} = 2f_1 - f_2$ mit $f_2 > f_1$ und einem optimalen Frequenzverhältnis von etwa $\dfrac{f_2}{f_1} = 1{,}2$ .

Bei geeigneter Wahl der Stimulusparameter (vorzugsweise $\dfrac{f_2}{f_1} = 1{,}2$ und $L_1 \geq L_2$) überlappen sich die von den Primärtönen ausgelösten Wanderwellen in der Cochlea in einem Bereich basal zum charakteristischen Abbildungsort der Frequenz $f_2$. Heutzutage werden üblicherweise DPOAE so angeregt, dass bei dem cochleären Abbildungsort des zweiten Primärtons beide Anregungsfrequenzen zu möglichst gleichgroßen Schwingungsamplituden der Basilarmembran führen, und dementsprechend werden diagnostische Schlüsse aus DPOAE-Befunden bei der Frequenz und entsprechend dem Anregungspegel des zweiten Primärtons $\{f_2, L_2\}$ interpretiert. DPOAE-Messungen können z.B. nach dem in der DE 102014108663 beschriebenen Verfahren bei verschiedenen Frequenzen durchgeführt und interpretiert werden.

In der Regel wird die Amplitude des DPOAE mittels Fouriertransformation aus dem Spektrum des gemessenen Signals extrahiert. Da die DPOAE einen sehr geringen Schalldruckpegel aufweisen, der deutlich unter der Hörschwelle liegen kann, muss ausreichend lange gemittelt werden, um einen gewissen Rauschabstand und damit eine zuverlässige diagnostische Aussage zu erhalten. Werden mehrere DPOAE bei einer Frequenz $f_2$ und verschiedenen Schalldruckpegeln $L_2$ gemessen und zu einer sogenannten Wachstumsfunktion zusammenfügt, ergibt sich eine genauere Aussage über die Funktion des cochleären Verstärkers im Innenohr. Für jede Anregungsfrequenz $f_2$ kann dann anhand der Wachstumskurve der sogenannte Schwellwert bestimmt werden, unter dem der geringste Pegel $L_2$ verstanden wird, bei dem das DPOAE noch einen gegebenen minimalen Rauschabstand erreicht. Diese Schwelle kann nicht gemessen werden, da das mitgemessene Rauschen endlich ist, sondern muss durch Extrapolation bestimmt werden.

Um eine diagnostische Aussage über den gesamten Frequenzbereich zu erhalten, werden daher typischerweise 6 bis 8 Wachstumsfunktionen sequentiell gemessen.

Die so bestimmten Wachstumskurven und daraus extrapolierten Schwellwerte können dann als Basis für eine verbesserte Diagnostik und zur Einstellung von Hörhilfen dienen, weil die extrapolierten Schwellwerte als unmittelbare Aussage über den Hörverlust anzusehen sind.

Der Generationsprozess der DPOAE und die bisher verwendeten Mess- und Auswerteverfahren sind auch in Dalhoff et al., "Schall- und Geschwindigkeits-DPOAE", in HNO 2010, 58: 543-555 beschrieben. Dort finden sich auch weitere Nachweise, auf die ausdrücklich verwiesen wird.

Die Anregung der DPOAE kann grundsätzlich sowohl durch kontinuierliche Töne als durch gepulste Töne erfolgen. Wie bereits vorhergehend erwähnt, versteht man dabei unter kontinuierlichen Tönen solche Töne, die so lange präsentiert werden, dass ihr Spektrum scharf ist.

Bei der gepulsten DPOAE wird zumindest einer der beiden Primärtöne (typischerweise der zweite Primärton $\{f_2, L_2\}$) gepulst eingespeist, während der andere Primärton (typischerweise der erste Primärton $\{f_1, L_1\}$) ebenfalls gepulst oder als Dauerton präsentiert wird. Das Verhältnis von $L_2$ zu $L_1$ wird zunächst in einem bestimmten Bereich eingestellt, anschließend wird $L_2$ schrittweise verändert. Bei den "gepulsten (Primär-)Tönen" handelt es sich nach der allgemeinen Fourier-Beziehung zwischen Zeit- und Frequenzbereich um Töne, deren Spektrum aufgrund der Kürze des Pulses verbreitert ist. Wird einer der Töne, wie z.B. vorerwähnter erste Primärton $\{f_1, L_1\}$ als Dauerton präsentiert, bedeutet das, dass der gepulst eingespeiste zweite Primärton $\{f_2, L_2\}$ während des Anhaltens des ersten Primärtons $\{f_1, L_1\}$ einen An- und Ausschaltprozess durchläuft. Andersherum kann auch der zweite Primärton $\{f_2, L_2\}$ als Dauerton präsentiert werden, während der gepulst eingespeiste erste Primärton $\{f_1, L_1\}$ während des Anhaltens des zweiten Primärtons $\{f_2, L_2\}$ einen An- und Ausschaltprozess durchläuft.

Verfahren der gepulsten DPOAE sind u.a. beschrieben in Dalhoff et al., "Two-source interference as the major reason for auditory-threshold estimation error based on DPOAE input-output functions in normal-hearing subjects", in Hearing Research 296 (2013), Seiten 67 - 82. Ein weiteres Verfahren der gepulsten DPOAE mit Pulsverschränkung ist in WO 2015/192969 A1 offenbart.

Dabei werden in dem in WO 2015/102969 A1 beschriebenen Verfahren ein erster gepulster Primärton $\{f_{1,1}, L_{1,1}\}$ und ein zweiter gepulster Primärton $\{f_{2,1}, L_{2,1}\}$ aufgebracht, wobei der $f_1$-Puls vor dem $f_2$-Puls einsetzt, und nach dem $f_2$-Puls endet, d.h. wobei der $f_1$-Puls länger ist als der $f_2$-Puls eines Pulspaares. WO 2015/102969 A1 bezieht sich dabei auf Kurzpulse von etwa 1 - 10 ms, deren Dauer etwa der Latenz zwischen Stimulation und Ausprägung des ersten nichtlinearen Distorsionsbeitrags einer DPOAE auf der Basilarmembran im Innenohr entspricht. In einer alternativen in WO 2015/102969 A1 beschriebenen Ausführungsform werden schließlich zwei gleich oder ähnlich lange Kurzpulse präsentiert, indem der $f_1$-Puls nach dem $f_2$-Puls einsetzt. Dabei werden zum Zweck der Zeitersparnis diese zwei Kurzpulse so abgestimmt, dass beide Pulse gleichzeitig am $f_2$-Abbildungsort im Innenohr eintreffen.

Alle bekannten DPOAE-Verfahren (einschließlich der oben beschriebenen gepulsten DPOAE) dienen zur Diagnose der Verstärkungsleistung des cochleären Verstärkers, welcher mit den äußeren Haarzellen (ÄHZ) als Schlüsselelement für eine mechanische, präneurale Verstärkung des Eingangssignals verantwortlich ist, und damit auch die Hörschwelle bis zu einem Hörverlust von etwa 50 bis 60 dB bestimmt (D. Zelle, E. Dalhoff, and A. W. Gummer, "Objektive Hördiagnostik mit DPOAE. Neue Erkenntnisse zur Generierung und klinischen Anwendung," HNO, vol. 64, no. 11, pp. 822-830, 2016 2016). Allerdings war es mittels der bekannten Verfahren bisher nicht möglich, auch die Ursache eines Verstärkungsverlusts zu bestimmen, insbesondere, ob dieser auf einen Verlust von intakten ÄHZ, oder vielmehr eine Beeinträchtigung der Energieversorgung durch die *Stria vascularis* (d.h. letztlich eine Reduzierung des endocochleären Potentials) verursacht wird.

Hier setzt das erfindungsgemäß weiterentwickelte Verfahren an, das sich die Aufgabe stellt, ein DPOAE Verfahren bereitzustellen, das die bekannten DPOAE Verfahren verbessern und das insbesondere Aufschluss über die Ursache eines Verstärkungsverlustes geben kann.

Erfindungsgemäß wird diese Aufgabe bei dem eingangs genannten Verfahren dadurch gelöst, dass ein Paar zweier Primärtöne mit je einer Anregungsfrequenz $f_1$ bzw. $f_2$ präsentiert wird, wobei der zweite Primärton $\{f_2, L_2\}$, der -entsprechend den üblichen Anregungsparadigmen- mit deutlich geringerem Schalldruck angeregt wird, zuerst präsentiert wird, während der erste Primärton $\{f_1, L_1\}$ mit einer zeitlichen Verzögerung $t_{lag}$ gegenüber dem zweiten Primärton $\{f_2, L_2\}$ präsentiert wird.

Der erste Primärton wird definiert durch eine erste Anregungsfrequenz $f_1$ und einen ersten Schalldruckpegel $L_1$. Er wird auch als "$f_1$-Primärton" bezeichnet. Der zweite Primärton wird definiert durch eine erste Anregungsfrequenz $f_2$ und einen ersten Schalldruckpegel $L_2$. Er wird auch als "$f_2$-Primärton" bezeichnet. Wenn im Folgenden von einem "ersten Primärton $\{f_1, L_1\}$" oder einem "$f_1$-Primärton" die Rede ist, beziehen sich die entsprechenden Ausführungen grundsätzlich sowohl auf den ersten Primärton des ersten Primärtonpaares des erfindungsgemäßen Verfahrens (d.h. den ersten Primärton $\{f_{1,1}, L_{1,1}\}$), als auch auf jeden weiteren ersten Primärton eines n-ten (weiteren) Primärtonpaares (d.h. den ersten Primärton $\{f_{1,n}, L_{1,n}\}$). Analog schließt der Begriff "zweiter Primärton $\{f_2, L_2\}$" oder "$f_1$-Primärton" grundsätzlich den zweiten Primärton $\{f_{2,1}, L_{2,1}\}$, als auch jeden weiteren zweiten Primärton eines n-ten (weiteren) Primärtonpaares (d.h. den zweiten Primärton $\{f_{2,n}, L_{2,n}\}$) mit ein.

Die zeitliche Verzögerung $t_{lag}$ bis zur Einspeisung des ersten Primärtons $\{f_1, L_1\}$ hängt typischerweise von den Latenzen der beiden beteiligten Wanderwellen ab. Latenzfunktionen für die nichtlineare Quelle sind in D. Zelle, A. W. Gummer, and E. Dalhoff, "Latencies of Extracted Distortion-Product Otoacoustic Source Components," in Mechanics of Hearing: Protein to Perception, vol. 1703, K. D. Karavitaki and D. P. Corey, Eds.: AIP conference proceedings, 2014 beschrieben.

Vorzugsweise beträgt $t_{lag}$ mindestens 0,5 Millisekunden (ms), d.h. der erste, vorzugsweise gepulste, Primärton $\{f_1, L_1\}$ wird vorzugsweise mindestens 0,5 ms nach dem zweiten Primärton $\{f_2, L_2\}$ eingeschaltet. Im Prinzip kann $t_{lag}$ beliebig groß gewählt werden, jedoch kann es von Vorteil sein, die zeitliche Verzögerung $t_{lag}$ möglichst gering zu halten, um die Messzeit nicht unnötig auszudehnen. Daher wird $t_{lag}$ vorzugsweise kleiner oder gleich 10 ms, kleiner oder gleich 7.5 ms oder besonders bevorzugt kleiner oder gleich 5 ms gewählt. Bevorzugt wird $t_{lag}$ aus dem Bereich zwischen 10 ms und 0 ms, besonders bevorzugt aus dem Bereich zwischen 5 ms und 0,5 ms ausgewählt.

Im erfindungsgemäßen Verfahren wird der erste Primärton $\{f_{1,1}, L_{1,1}\}$ vorzugsweise gepulst präsentiert (d.h. als "$f_1$-Puls"). Wie oben angegeben, werden unter "gepulsten" Primärtonen Töne verstanden die nach der allgemeinen Fourier-Beziehung zwischen Zeit- und Frequenzbereich ein aufgrund der Kürze des Pulses verbreitertes Spektrum aufweisen. Weiterhin kann auch der zweite Primärton $\{f_{2,1}, L_{2,1}\}$ gepulst (d.h. als "$f_2$-Puls") oder als Dauerton präsentiert werden. "Gepulste" Primärtone durchlaufen während der Messdauer einen An- und Abschaltprozess und werden typischerweise für eine Dauer von 50 ms oder weniger präsentiert. Im erfindungsgemäßen Verfahren wird der zweite Primärton $\{f_{2,1}, L_{2,1}\}$ als Dauerton oder gepulst ("$f_2$-Puls") präsentiert, während der erste Primärton $\{f_{1,1}, L_{1,1}\}$ vorzugsweise gepulst eingespeist wird ("$f_1$-Puls"). Vorzugsweise werden beide Primärtöne gepulst präsentiert, wobei die Pulslänge des zweiten Primärtons $\{f_{2,1}, L_{2,1}\}$ die Pulslänge des ersten Primärton $\{f_{1,1}, L_{1,1}\}$ übertrifft.

Weiterhin kann es unabhängig von der Art der Präsentation des zweiten Primärtons als Dauerton oder Puls bevorzugt sein, dass der $f_1$-Puls während des Anhaltens des zweiten Primärtons $\{f_{2,1}, L_{2,1}\}$ einen An- und Abschaltprozess durchläuft. Werden beide Primärtöne des Primärtonpaares also gepulst (mit anderen Worten: als $f_1$-Puls bzw. der $f_2$-Puls) präsentiert, kann es bevorzugt sein, dass die Pulslänge des $f_1$-Pulses kürzer ist als die Pulslänge des $f_2$-Pulses.

Alternativ kann bei gepulster Präsentation beider Primärtöne der zweite gepulste Primärton $\{f_{2,1}, L_{2,1}\}$ zuerst eingeschaltet werden, wohingegen der erste gepulste Primärton $\{f_{1,1}, L_{1,1}\}$ mit einer zeitlichen Verzögerung $t_{lag}$ (wie oben definiert) präsentiert und *nach* dem zweiten gepulsten Primärton $\{f_{2,1}, L_{2,1}\}$ abgeschaltet wird. Dieses Verfahren kann auch als eine Mischung des Anregungsschemas nach PTA-I und PTA-II betrachtet werden. Die Präsentation erfolgt dabei zunächst nach dem Anschalten entsprechend PTA-II. Vorzugsweise liefert die DPOAE-Anregung gem. PTA-II Hinweise auf die korrekte Homöostase des Innenohrs. Vor dem Abschalten erfolgt die Präsentation entsprechend PTA-I, wodurch der Interferenzzustand der nichtlinearen und kohärenten Reflexionsquelle bestimmt werden kann.

Der $f_1$-Puls des ersten Primärtonpaares $\{f_{1,1}, L_{1,1}; f_{2,1}, L_{2,1}\}$ kann also vor oder nach dem Ende des $f_2$-Pulses des ersten Primärtonpaares abgeschaltet werden.

In jedem Fall kann die Dauer des $f_1$-Pulses und/oder die Dauer des $f_2$-Pulses im PTA-II Anregungsschema größer als die Latenz der evozierten DPOAE gewählt werden, vorzugsweise mindestens doppelt, noch bevorzugter mindestens dreimal und am bevorzugtesten mindestens fünfmal so lang. Es kann besonders bevorzugt sein, die Dauer des $f_1$-Pulses und/oder die Dauer des $f_2$-Pulses im PTA-II Anregungsschema so zu wählen, dass sie das fünf- bis siebenfache der Latenz der evozierten DPOAE entspricht. Die Latenz der evozierten DPOAE hängt von der gewählten Frequenz für jeden $f_1$- und $f_2$-Primärton ab (siehe Zelle et al. AIP Conference Proceedings 1703, 090023 (2016)).

Dabei kann die Länge des ersten Primärtons $\{f_{1,1}, L_{1,1}\}$ vorzugsweise zwischen 40 ms bis 1 ms, noch bevorzugter zwischen 30 ms und 2 ms, noch mehr bevorzugt zwischen 25 ms und 2 ms betragen. Die Länge des zweiten Primärtons $\{f_{2,1}, L_{2,1}\}$ wird dann dementsprechend länger gewählt. So kann der zweite Primärton $\{f_{2,1}, L_{2,1}\}$ als Dauerton oder gepulst präsentiert werden. Abhängig vom gewählten Gesamt-Anregungsmuster kann die bevorzugte Pulslänge jedoch unter Umständen davon abweichen. Insbesondere für das kombinierte PTA-I/PTA-II Anregungsschema kommen wie unten beschrieben ggf. auch längere Pulse von 200 ms oder weniger, 100 ms oder weniger oder 50 ms oder weniger in Betracht.

Unter der "Dauer" oder "Länge" eines Pulses (auch als "Pulslänge" oder "Pulsbreite" bezeichnet) die sogenannte volle Halbwertsbreite ($T_{HB}$ bzw. "full width half maximum" ($T_{FWHM}$) verstanden. Für diese Pulslänge wird die Zeit bestimmt, ab der die kosinusförmige Anstiegsflanke auf die Hälfte des Gleichgewichtswerts ("steady-state") angestiegen ist, bis zu dem entsprechenden Zeitpunkt in der Abschaltflanke. Diese Pulslänge ergibt sich aus der bevorzugten Pulsform, gemäß der die Pulse einen kosinusförmigen Anstieg von typischerweise 0,1 ms bis 4 ms Länge, einen "steady-state" mit dem Schalldruckpegel $L_2$ bzw. $L_1$, der typischerweise 2 ms bis 12 ms lang ist, gefolgt von einem weiteren kosinusförmigen Abschnitt.

Die Schalldruckpegel $L_1$ und $L_2$ des mindestens einen Primärtonpaares können vorzugsweise ähnlich gewählt werden, da mit zunehmender Schalldruckpegel-Differenz die Gefahr der gegenseitigen Suppression zunehmen kann. Werden mehrere Primärtonpaare in einer Messung präsentiert, kann für die maximale Schalldruckpegel-Differenz zwischen den $L_2$-Schalldruckpegeln von beispielsweise vier Primärtonpaaren eine feste Schwelle definiert werden, die beispielsweise zwischen 5 und 15 dB liegen kann.

Diese hier beschriebenen Anregungsmuster mit einer (um $t_{lag}$) zeitversetzten Präsentation des (vorzugsweise gepulsten) $f_1$-Primärtons werden insgesamt auch als Primärton-Arrangement II (PTA-II) bezeichnet und steht im Gegensatz zu im Stand der Technik bekannten Verfahren, bei denen der $f_1$-Primärton zuerst präsentiert wird, und der $f_2$-Primärton zeitversetzt einsetzt. Solcherart aus dem Stand der Technik bekannte Anregungsmuster werden hier auch als Primärton-Arrangement I (PTA-I) bezeichnet.

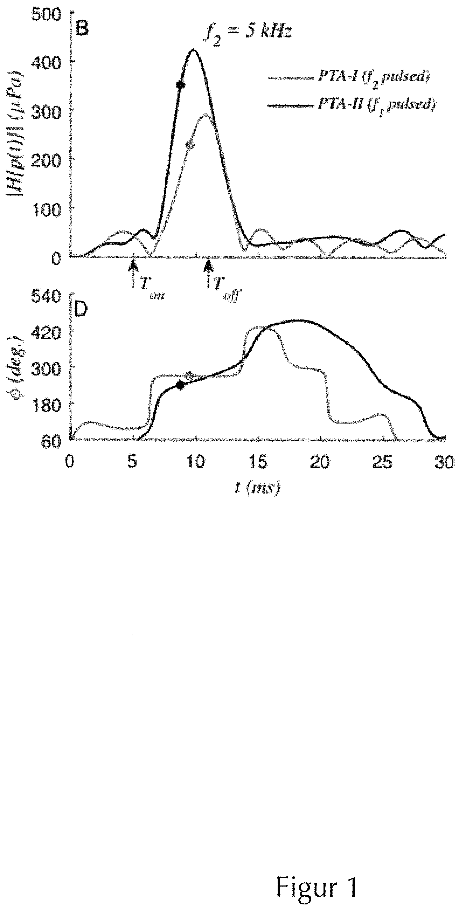

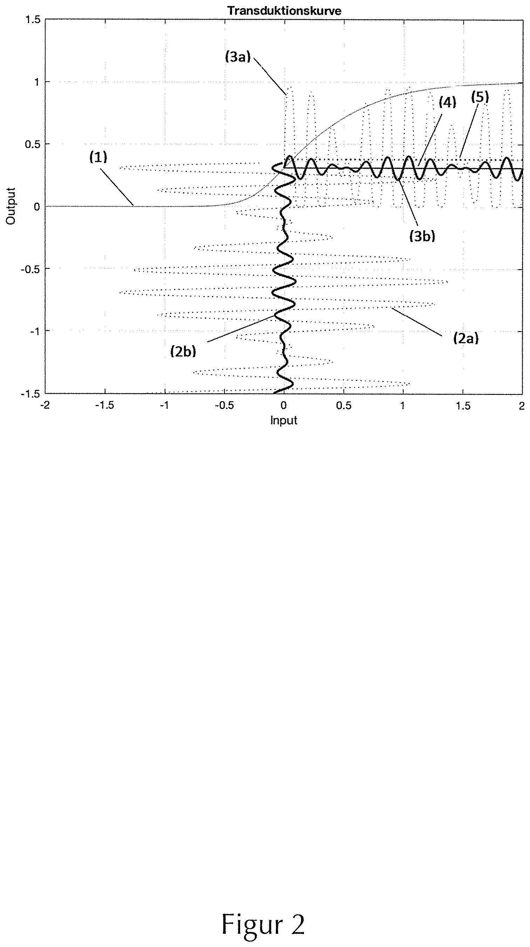

Die Erfinder erkannten, dass eine DPOAE-Anregung gemäß PTA-I bzw. PTA-II zu unterschiedlichen Amplituden führen (vgl. Abb. 1). Insbesondere ist die Antwort der nichtlinearen Quelle (d.h. des zeitlich zuerst kommenden Teils der Einhüllenden des $2f_1$-$f_2$-DPOAE-Zeitsignals) für PTA-II größer als für PTA-I. Diese ist besonders interessant, da ihre Amplitude meistens größer ist als die der kohärenten Reflexionsquellen, und sie im Gegensatz dazu von keinem zusätzlichen Prozess (nämlich der vermuteten Rauigkeit der Impedanzfunktion) abhängig ist. Die Ursache für die beobachtete Abweichung hinsichtlich der Antwort der nichtlinearen Quelle liegt in der Asymmetrie der sogenannten mechano-elektrischen Transduktionskurve der ÄHZ. Diese Transduktionskurve stellt die Abhängigkeit des intrazellulären Rezeptorpotentials (Output) in Abhängigkeit des Stimulus, bspw. der Auslenkung der Stereozilien der ÄHZ (Input), dar. Der Arbeitspunkt liegt bei intakter Homöostase der Cochlea etwa am Punkt der maximalen Steigung der Kurve (vgl. vereinfachte Stimulation in Abb. 2).

Die Anregung der nichtlinearen Quelle der DPOAE findet in der Nähe des Abbildungsortes des zweiten Primärtons mit der Frequenz $f_2$ ("$f_2$-Abbildungsort") statt, wo sich die Wanderwellen beider Primärtöne am stärksten überlappen. Entscheidend für die Generierung der DPOAE ist der Bereich von etwa ½ Oktave basal bis zum $f_2$-Abbildungsort, da hier die ÄHZ maximal zur Verstärkung der Eingangsschwingungen beitragen. Am $f_2$-Abbildungsort haben beide Primärtöne unterschiedliche Latenzen, da die Phasenübertragungsfunktion einer cochleären Wanderwelle zum Abbildungsort hin immer steiler wird. Daher findet die größte Phasendrehung erst kurz vor dem Maximum der Wellenamplitude statt. Da die Wanderwelle des ersten Primärtons ("$f_1$-Wanderwelle") am $f_2$-Abbildungsort ihr Amplitudenmaximum noch nicht erreicht hat, ist ihre Latenz an diesem Ort deutlich geringer. Werden beide Primärtöne gleichzeitig angeschaltet, wird die $f_1$-Wanderwelle den $f_2$-Abbildungsort zuerst erreichen und bereits zur Verlagerung des DC-Potentials beitragen, bevor der zweite Primärton $\{f_2, L_2\}$ eintrifft und das DPOAE erzeugen werden kann. Dies ist im PTA-I-Schema der Fall.

Bei Anwendung des erfindungsgemäßen PTA-II-Schemas -wobei der (gepulste) zweite Primärton $\{f_2, L_2\}$ zuerst ein- und optional zuletzt ausgeschaltet wird - führt der relativ leisere $f_2$-Puls noch zu keiner merklichen DC-Potentialverschiebung, so dass der gepulste erste Primärton $\{f_1, L_1\}$ präsentiert wird, wenn der cochleäre Verstärker sich noch (fast) im Ruhezustand befindet. Bei normalem physiologischen Zustand der Cochlea befindet sich dann der Arbeitspunkt nahe dem Optimum, d.h. der höchsten Verstärkungsleistung. Man erhält also eine höhere DPOAE-Amplitude.

Vorteilhafterweise kann daher insbesondere durch den Vergleich zweier Messungen, also einer PTA-I und eine PTA-II-Messung bei derselben Frequenz und denselben Anregungspegeln der beiden Primärtöne, festgestellt werden, ob die erwartete Änderung der Amplitude vorliegt und daher von einer optimalen, nichtpathologischen Einstellung des Arbeitspunkts des cochleären Verstärkers ausgegangen werden kann.

Im erfindungsgemäßen Verfahren erfolgt die Anregung mit zumindest einem Paar von Primärtönen $\{f_{1,1}, L_{1,1}\}$ und $\{f_{2,1}, L_{2,1}\}$, die vorzugsweise jeweils beide gepulst (d.h. als "Pulspaar") präsentiert werden.

Die Anregungsfrequenzen $f_1$ und $f_2$ eines Primärtonpaares sind dabei vorzugsweise über ein Frequenzverhältnis $f_2/f_1 = 1{,}2$ verknüpft. Abweichend davon kann dieses Frequenzverhältnis jedoch auch auf jeden anderen geeigneten Wert, vorzugsweise zwischen 1,15 und 1,35 festgelegt werden, siehe z.B. Johnson et al. "Influence of primary-level and primary-frequency ratios on human distortion product otoacoustic emissions", in J. Acoust. Soc. Am. 119, 2006, Seiten 418 - 428.

Solche Primärtonpaare bestehen üblicherweise aus erstem (gepulsten) und zweitem (optional gepulstem) Primärton $\{f_1, L_1\}$ und $\{f_2, L_2\}$, bei denen die Frequenz $f_1$ über ein definiertes Frequenzverhältnis aus $f_2$ bestimmt wird. Ebenso kann der Schalldruckpegel $L_1$ nach einer vordefinierten Regel aus $L_2$ berechnet werden. Der Begriff "Primärtonpaare" schließt den Begriff der "Pulspaare" ein, welcher Paare aus erstem gepulsten und zweitem gepulstem Primärton $\{f_1, L_1\}$ und $\{f_2, L_2\}$ bezeichnet.

Wie oben beschrieben, werden im erfindungsgemäßen Verfahren der erste und der zweite Primärton eines Primärtonpaares geringfügig zeitversetzt präsentiert, wobei der (vorzugsweise gepulste) zweite Primärton $\{f_{2,1}, L_{2,1}\}$ vor dem (vorzugsweise gepulsten) ersten Primärton $\{f_{1,1}, L_{1,1}\}$ einsetzt. Endet der (vorzugsweise gepulste) erste Primärton $\{f_{1,1}, L_{1,1}\}$ vor dem (vorzugsweise gepulsten) zweiten Primärton, und insbesondere wenn bei gepulste Präsentation beider Primärtöne der erste Primärton $\{f_{1,1}, L_{1,1}\}$ eine kürzere Pulslänge als der zweite Primärton $\{f_{2,1}, L_{2,1}\}$ aufweist, wird dieses Anregungsschema auch als "PTA-II" bezeichnet. Andersherum wird ein Anregungsschema, bei dem ein (vorzugsweise gepulster) erster Primärton $\{f_{1,n}, L_{1,n}\}$ vor dem (vorzugsweise gepulsten) zweiten Primärton $\{f_{2,n}, L_{2,n}\}$ einsetzt und nach diesem endet, insbesondere wenn bei gepulster Präsentation beider Primärtöne der zweite Primärton $\{f_{2,n}, L_{2,n}\}$ eine kürzere Pulslänge als der erste Primärton $\{f_{1,n}, L_{1,n}\}$ aufweist, als "PTA-I" bezeichnet. Typische Pulslängen sind weiter oben genannt und grds. auf PTA-I und PTA-II anwendbar.

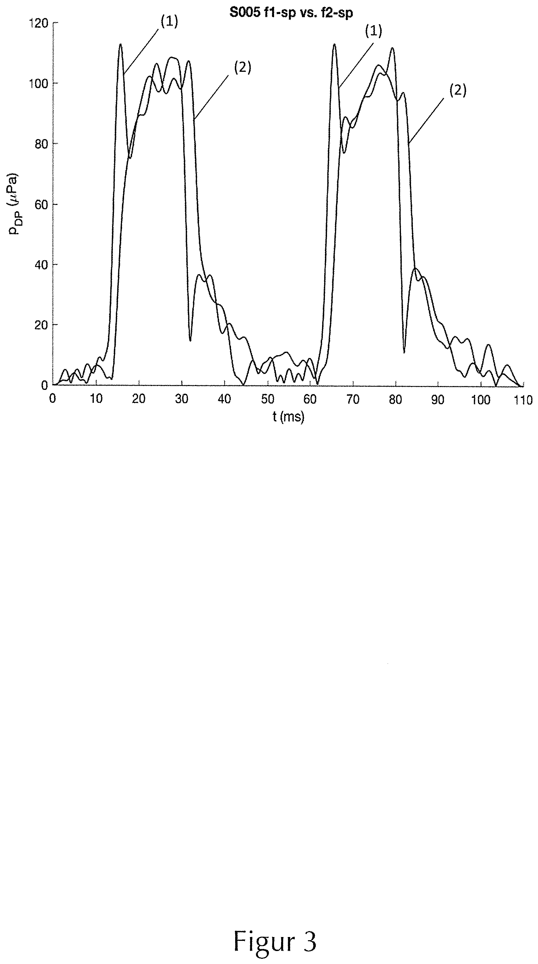

Es kann erfindungsgemäß bevorzugt sein, die Länge des $f_1$-Pulses über die Latenz der nichtlinearen Quelle hinaus zu verlängern ("verlängerter $f_1$-Puls"), um vorzugsweise die stimulusbedingte Verschiebung des Rezeptorpotentials in einer einzelnen Messung gezeigt werden (vgl. Abb. 3). Typische Pulslängen für verlängerte $f_1$-Pulse liegen vorzugsweise zwischen 5 und 20 ms.

Wie oben beschrieben, setzt im erfindungsgemäßen Verfahren der $f_2$-Primärton eines Primärtonpaares ein, bevor der (vorzugsweise gepulste) $f_1$-Primärton einsetzt, und kann enden, <u>*nachdem*</u> der (gepulste) $f_1$-Primärton beendet

wurde, d.h. der $f_2$-Primärton kann länger als der (gepulste) $f_1$-Primärton eines Primärtonpaares andauern. Wird der $f_1$-Primärton mit einer Zeitverzögerung $t_{lag}$ gegenüber dem $f_2$-Primärton eingeschaltet, so erreicht die DPOAE vorzugsweise zu Beginn ein Maximum und fällt dann ab, um wiederum anzusteigen (vgl. Abb. 3). Dieses nachfolgende Ansteigen kann prinzipiell durch den Einfluss der zweiten Quelle (Reflexionskomponente) entstehen, aber auch durch langsamere Regelungsprozesse der Differenz zwischen intra- und extrazellulären Potentialen, bzw. durch die Dynamik der örtlich begrenzten positiven Rückkopplungsschleife, die zu der cochleären Verstärkung führt.

Beim Ausschalten des (vorzugsweise gepulsten) $f_1$-Primärtons findet ein Erholungsprozess statt, der prinzipiell sowohl durch die Wiederherstellung des Ruhepotentials als auch durch Einflüsse der zweiten, also der kohärenten Reflexionsquelle resultieren kann.

Bei im Stand der Technik bekannten Verfahren mit einer Anregung gemäß PTA-I wird nach Abschalten des (typischerweise gepulsten) $f_2$-Primärtons dagegen weder das frühe Maximum, noch der Erholungsprozess erreicht. Dagegen kann eine einzelne Messung gemäß dem erfindungsgemäßen Verfahren mit einem (vorzugsweise gepulsten) $f_1$-Primärton bereits das Vorhandensein einer DC-Potentialverschiebung abklären. Liegt eine pathologische Verschiebung des DC-Potentials vor, wird erwartet, dass die Änderung je nach Frequenz deutlich geringer ausfällt, und unter Umständen (etwa bei niedrigen Frequenzen) sogar das Vorzeichen wechselt.

Alternativ setzt im erfindungsgemäßen Verfahren der $f_2$-Primärton eines Primärtonpaares ein, bevor der (vorzugsweise gepulste) $f_1$-Primärton einsetzt, und kann enden, _bevor_ der $f_1$-Primärton beendet wurde. In anderen Worten endet damit der $f_1$-Puls nach dem Abschalten des $f_2$-Pulses.

Das erfindungsgemäße Verfahren kann weiterhin die Ausgabe mindestens eines weiteres (n-ten) Primärtonpaares aus je einem ersten Primärton mit Frequenz $f_{1,n}$ und Schalldruckpegel $L_{1,n}$ und einem zweiten Primärton mit Frequenz $f_{2,n}$ und Schalldruckpegel $L_{2,n}$ umfassen, wobei $f_{2,n} > f_{1,n}$. Das weitere Primärtonpaare weist dabei eine $f_2$-Anregungsfrequenz auf, die sich entweder von der $f_2$-Anregungsfrequenz des ersten Primärtonpaares unterscheidet (d.h. $f_{2,1} \neq f_{2,n}$) oder eine $f_2$-Anregungsfrequenz, die der $f_2$-Anregungsfrequenz des ersten Primärtonpaares entspricht (d.h. $f_{2,1} = f_{2,n}$). Die Präsentation des mindestens einen weiteren Primärtonpaares kann entweder vor oder nach der Präsentation des ersten Primärtonpaares erfolgen. Es ist auch möglich, dass die Präsentation des ersten Primärtonpaares in die Einspeisung weiterer Primärtonpaare "eingebettet" ist.

Das mindestens eine weitere Primärtonpaar kann gemäß dem Anregungsmuster PTA-II (wie das erste Primärtonpaar) oder gemäß dem Anregungsmuster PTA-I präsentiert werden. Das bedeutet, (a) der zweite Primärton des mindestens einen weiteren Primärtonpaares $\{f_{2,n}, L_{2n}\}$ kann mit einer zeitlichen Verzögerung $t_{lag}$ nach dem ersten Primärton $\{f_{1,n}, L_{1,n}\}$ dieses mindestens einen weiteren Primärtonpaares ausgegeben werden, oder (b) der erste Primärton des mindestens einen weiteren Primärtonpaares $\{f_{1,n}, L_{1,n}\}$ kann mit einer zeitlichen Verzögerung $t_{lag}$ nach dem zweiten Primärton $\{f_{2,n}, L_{2,n}\}$ dieses mindestens einen weiteren Primärtonpaares ausgegeben werden.

Dabei kann n aus jeder beliebigen, vorzugsweise ganzen, Zahl $\geq 2$ ausgewählt werden. Vorzugsweise ist n = 2.

Dabei entspricht Option (a) vorzugsweise einer Anregung gemäß PTA-I. Hierbei ist insbesondere vorgesehen, dass der zweite Primärton und optional der erste Primärton gepulst präsentiert wird. Werden beide Primärtone gepulst präsentiert, kann die Pulslänge des $f_2$-Pulses vorzugsweise kürzer als die Pulslänge des $f_1$-Pulses sein.

Option (b) entspricht vorzugsweise einer Anregung gemäß PTA-II. Hierbei ist insbesondere vorgesehen, dass der erste Primärton und optional der zweite Primärton gepulst präsentiert wird. Werden beide Primärtone gepulst präsentiert, kann die Pulslänge des $f_1$-Pulses vorzugsweise kürzer als die Pulslänge des $f_2$-Pulses sein.

Wie oben beschrieben, ist insbesondere ein kombiniertes PTA-I/PTA-II Anregungsschema sinnvoll, um die Änderung der erhaltenen Amplitude der angeregten DPOAE (insbesondere der nichtlinearen Quelle) miteinander zu vergleichen. Dazu wird vorzugsweise nach der erfindungsgemäßen Anregung nach dem PTA-II Schema mindestens ein weiteres n-tes Primärtonpaar $\{f_{1,n}, L_{1,n}, f_{2,n}, L_{2n}\}$ ausgegeben, wobei der zweite Primärton $\{f_{2,n}, L_{2n}\}$ des mindestens einen weiteren n-ten Primärtonpaares mit einer zeitlichen Verzögerung $t_{lag}$ nach dem ersten Primärton $\{f_{1,n}, L_{1,n}\}$ dieses Primärtonpaares ausgegeben wird. Dabei kann die Ausgabe des mindestens einen weiteren n-ten Primärtonpaares $\{f_{1,n}, L_{1,n}, f_{2,n}, L_{2n}\}$ optional vor oder nach der Ausgabe des ersten Primärtonpaares $\{f_{1,1}, L_{1,1}, f_{2,1}, L_{2,1}\}$ erfolgen.

Bevorzugt kann das erfindungsgemäße Verfahren - insbesondere dann, wenn es die Stimulation mittels PTA-I und PTA-II umfasst-weiterhin einen Schritt des Vergleichs der durch Ausgabe des ersten Primärtonpaares $\{f_{1,1}, L_{1,1}, f_{2,1}, L_{2,1}\}$ evozierten DPOAE mit denen durch die Ausgabe eines weiteren Primärtonpaares $\{f_{1,n}, L_{1,n}, f_{2,n}, L_{2,n}\}$ mit identischen Schalldruckpegeln L und Frequenzen $f$, jedoch unterschiedlicher Verzögerung zwischen den Stimuluspulsen umfassen.

Wie oben beschrieben können gemäß dem erfindungsgemäßen Verfahren der erste Primärton $\{f_{1,1}, L_{1,1}\}$ und/oder $\{f_{1,n}, L_{1,n}\}$ ) und optional der zweite Primärton $\{f_{2,1}, L_{2,1}\}$ und/oder $\{f_{2,n}, L_{2n}\}$ vorzugsweise gepulst, d.h. als "$f_1$-Puls" bzw. "$f_2$Puls" präsentiert werden. Dies gilt sowohl für eine Anregung nach PTA-II als auch für eine mögliche zusätzliche Anregung nach PTA-I.

Für die bevorzugten Anregungsfrequenzen, Schalldruckpegel, Pulslängen und weitere Parameter gelten für die Anregung nach PTA-I grundsätzlich die Ausführungen in Bezug auf PTA-II entsprechend.

Insbesondere kann die Dauer des $f_1$-Pulses und/oder die Dauer des $f_2$-Pulses im PTA-I Anregungsschema größer als die Latenz der evozierten DPOAE gewählt werden, vorzugsweise mindestens doppelt, noch bevorzugter mindestens dreimal und am bevorzugtesten mindestens fünfmal so lang. Es kann besonders bevorzugt sein, die Dauer des $f_1$-Pulses und/oder die Dauer des $f_2$-Pulses im PTA-I Anregungsschema so zu wählen, dass sie das fünf- bis siebenfache der Latenz der evozierten DPOAE entspricht.

Insbesondere kann die Dauer des $f_1$-Pulses $\{f_{1,1}, L_{1,1}\}$ des ersten Primärtonpaares und/oder des $f_2$-Pulses $\{f_{2,n}, L_{2,n}\}$ des n-ten weiteren Primärtonpaares 200 ms oder weniger, 100 ms oder weniger, 50 ms oder weniger, zwischen 40 ms bis 1 ms, zwischen 30 ms und 2 ms oder zwischen 25 ms und 5 ms betragen. Besonders für ein kombiniertes PTA-I/PTA-II Anregungsschema kann dabei die Pulslänge auf 200 ms oder weniger, 100 ms oder weniger oder 50 ms oder weniger verlängert werden. Weiterhin kann die Pulslänge des $f_2$-Pulses des n-ten weiteren Primärtonpaares $\{f_{2,n}, L_{2,n}\}$ vorzugsweise kürzer sein als die Pulslänge des $f_1$-Pulses des n-ten weiteren Primärtonpaares $\{f_{1,n}, L_{1,n}\}$.

Wie weiter oben erklärt, kann die Kombination einer Anregung gemäß PTA-II mit dem ersten Primärtonpaar $\{f_{1,1}, L_{1,1}, f_{2,1}, L_{2,1}\}$ mit einer Anregung gemäß PTA-I mit einem zweiten Primärtonpaar $\{f_{1,n}, L_{1,n}, f_{2,n}, L_{2,n}\}$ vorteilhaft sein, um die erhaltenen Amplituden miteinander zu vergleichen und Rückschlüsse auf die Einstellung des Arbeitspunktes des cochleären Verstärkers zu ziehen. Dazu werden die Anregungsfrequenzen und Schalldruckpegel zweckmäßigerweise gleich gewählt, und nur das jeweilige Anregungsschema geändert, um die Vergleichbarkeit der erhaltenen Ergebnisse zu garantieren. In anderen Worten sind Frequenz und Schalldruckpegel des ersten Primärtons des ersten und jeden weiteren n-ten Primärtonpaares und/oder Frequenz und Schalldruckpegel des zweiten Primärtons des ersten und jeden weiteren n-ten Primärtonpaares vorzugsweise identisch. Noch anders ausgedrückt gilt in den erfindungsgemäßen Verfahren bevorzugt: $\{f_{1,1}, L_{1,1}\} = \{f_{1,n}, L_{1,n}\}$ und/oder $\{f_{2,1}, L_{2,1}\} = \{f_{2,n}, L_{2,n}\}$, noch bevorzugter $\{f_{1,1}, L_{1,1}\} = \{f_{1,n}, L_{1,n}\}$ und $\{f_{2,1}, L_{2,1}\} = \{f_{2,n}, L_{2,n}\}$,

Zusammenfassend besteht die erfindungsgemäße Leistung unter anderem darin, ein gegenüber dem Stand der Technik neues Anregungsschema einzusetzen, wobei vorzugsweise (1) zwei unterschiedliche Messungen gemäß PTA-II und PTA-I (wobei für die Wahl der Anregungsfrequenzen und Schalldruckpegel das oben gesagte gilt) oder (2) eine Messung gemäß PTA-II mit verlängertem $f_1$-Puls durchgeführt wird.

Eine entscheidende Neuerung besteht also gemäß Möglichkeit (1) darin, dass für ein gegebenes Primärtonpaar die DPOAE-Antwort im Hinblick auf zwei unterschiedliche Anregungsformen (PTA-I und PTA-II) miteinander verglichen werden. Diese Möglichkeit ist im Stand der Technik nicht vorgesehen. Die Stimulation mittels unterschiedlicher Anregungsformen kann die DPOAE-Antwort im Prinzip auf drei unterschiedliche Weisungen beeinflussen:

Suppression: Im Innenohr führt die kompressive Nichtlinearität der Übertragungskurve der mechanoelektrischen Transduktion, gekennzeichnet durch die Grenzzustände vollständig geöffneter bzw. geschlossener Ionenkanäle, dazu, dass die Präsentation eines zweiten Signals - also hier das Anschalten des jeweils zeitverzögerten Primärtons - zu sogenannten Suppressionseffekten führt; und zwar zwangsläufig, denn DPOAE werden vorzugsweise dann erzeugt, wenn beide Primärtöne gleichzeitig am $f_2$-Abbildungsort eintreffen. Im Innenohr ist die Wanderwelle, die durch einen einzelnen Primärton hervorgerufen wird, stark abhängig von einem Gebiet, das sich etwa 1/3 einer Oktave basal ihres Maximums erstreckt. In diesem Gebiet wird durch die äußeren Haarzellen der Wanderwelle die zusätzliche Energie phasenrichtig zugeführt, welche zu der cochleären Verstärkung führt. Da beide Primärtöne so gewählt werden, dass sie am $f_2$-Abbil- dungsort etwa dieselbe Amplitude erreichen (also der $f_1$-Primärton deutlich stärker anregt, da er ja weiter apikal sein Maximum bildet), stimuliert der $f_1$-Primärton mit seiner flacheren basalen Flanke die äußeren Haarzellen im Gebiet etwa 1/3 Oktave stärker als der $f_2$-Primärton (welcher am $f_2$-Abbildungsort ein relativ scharfes Maximum ausbildet). Daher fallen die DPOAE-Anworten je nach Anregungsparadigma (PTA-I oder PTA-II) unterschiedlich aus, weil die basal zum $f_2$-Abbildungsort liegenden äußeren Haarzellen im Fall einer Erstanregung mit dem $f_1$-Primärton gemäß PTA-I stärker saturiert werden und bei der Generierung des folgenden $f_2$-Primärtons nicht mehr die volle Verstärkung zur Verfügung stellen können.

DC-Verschiebung: DC-Effekte sind die Effekte einer vergleichsweise niederfrequenten, transienten Verlagerung des Ruhepotentials in den äußeren Haarzellen, und damit auch der Lage der Basilarmembran und auch der Tektorialmembran. Bei normaler physiologischer Homöostase im Innenohr befindet sich der Arbeitspunkt relativ nahe dem Zustand vollständig geschlossener Ionenkanäle (also deutlich asymmetrisch). Bei hohen Amplituden, bei denen das Eingangssignal durch beide Grenzzustände beschnitten wird, sollte der tiefpaßgefilterte Signalanteil (also die transiente Ruhepotentialverlagerung), in das Mittelpotential übergehen. Wird der $f_1$-Primärton gemäß PTA-I zuerst eingeschaltet, hat dieser Übergang zum Mittenpotential in den meisten Zellen stattgefunden, bevor der $f_2$-Primärton eingeschaltet wird und eine DPOAE-Antwort gemessen werden konnte. Daher wird die Verlagerung des Ruhepotentials, die zu einer geringfügigen Verringerung der Verstärkung der äußeren Haarzellen führt, nicht sichtbar. Im PTA II-Arrangement trifft dagegen der $f_2$-Primärton zuerst ein, und im Gebiet entsprechend 1/3 Oktave basal zum $f_2$-Abbildungsort findet die Ruhepotentialverlagerung in vollem Umfang erst statt, sobald der $f_2$-Primärton angeschaltet wird. Diese Ruhepotentialverlagerung wird daher sichtbar, indem die DPOAE-Antwort zuerst eine höhere Amplitude erreicht, sich dann aber aufgrund der Ruhepotentialverlagerung verringert. Ab diesem Zeitpunkt verlaufen die DPOAE-Antworten beider Anregungsparadigmen fast identisch, bis im Abschalten ein ähnlicher Effekt zu beobachten ist (Aufhebung des verlagerten Ruhepotentials). Damit erklärt sich auch der markante Unterschied beider DPOAE-Kurven: Im PTA II-Anregungsschema steigt die DPOAE-Antwort im Vergleich zum PTA-I Anregungsschema ca. 2 ms eher an. Dieser Wert entspricht ca. 1/3 der Latenz der DPOAE-Antworten für die Frequenz von $f_2$=2 kHz (ca. 6 ms). Der später eingeschaltete $f_1$-Primärton erreicht den weiter basal liegenden $f_2$-Abbildungsort deutlich bevor die DPOAE-Antwort ihr Maximum ausbildet, und ist am $f_2$-Abbildungsort noch nicht deutlich bandbegrenzt. Breitbandige Signale führen im Allgemeinen zu steileren Flanken im Zeitbereich.

Efferente Innervation der äußeren Haarzellen. Ein Ton erzeugt im gesunden Ohr einen neuralen Reiz, der von den inneren Haarzellen über den Hirnstamm zum Cortex aufsteigt. Im Hirnstamm koppelt Interneurone den Reiz über eine efferente Innervation auf die äußeren Haarzellen zurück, und führen im gesunden Ohr zu einer schwachen Dämpfung des cochleären Verstärkers. Dieser Effekt hat aber in der Regel höchsten eine Dämpfung von 1 dB zur Folgen (<10% Amplitudenreduktion), zweitens liegt die Zeitkonstante dieses Effekts bei etwa 40 ms, kann also nach gegenwärtigem Kenntnisstand nicht zu dem Unterschied zwischen den beiden Pulsantwortformen führen.

Werden in dem erfindungsgemäßen Verfahren in einer Messung mehrere Primärtonpaare (gemäß PTA-I oder PTA-II) präsentiert, kann die Anwendung des unten beschriebenen Pulsverschränkungsverfahrens zweckmäßig sein, um die Messdauer zu verkürzen. Dazu wird ein Satz aus dem ersten Primärtonpaar $\{f_{1,1}, L_{1,1}; f_{2,1}, L_{2,1}\}$ und dem mindestens einem weiteren Primärtonpaar $\{f_{1,n}, L_{1,n}; f_{2,n}, L_{2,n}\}$ in einem Block ausgegeben, der während der Messdauer mehrfach wiederholt wird.

Vorteilhafterweise kann das erfindungsgemäße Verfahren einen weiteren, vorgeschalteten Schritt enthalten, mit dem zu Beginn der Messungen überprüft wird, ob die Frequenz $f_{dp}$ einer der evozierten DPOAE mit einer spontanen Emission (SOAE) interferiert. Dies ist insbesondere von Vorteil, wenn während der Messung mehrere Primärtonpaare präsentiert werden sollen (zum Beispiel nach dem o.g. Pulsverschränkungsverfahren). Damit können Artefakte und Störquellen schon zu Beginn der Messung erkannt werden. Ist dies der Fall, kann beispielsweise die Blockzeit $T_B$ bzw. die Zeitdauer $T_S$ für einen oder alle Slots so angepasst werden, dass die Abklingzeit des DPOAE so weit verlängert wird, dass sein Schalldruckpegel unter eine gewisse Schwelle gelangt, bevor das nächste Primärtonpaar präsentiert wird. Alternativ kann beispielsweise die $f_2$-Anregungsfrequenz verschoben werden, um einen Mindestabstand zu der SOAE herzustellen.

Vorzugsweise kann zu diesem Zweck zu Beginn einer Messung für ein Primärtonpaar mit einem ersten Primärton $\{f_1, L_1\}$ und einem zweiten Primärton $\{f_2, L_2\}$ ein DPOAE gemessen werden. Für den Fall, dass kein DPOAE messbar ist, können die Schalldruckpegel $L_2$ und $L_1$ inkremental erhöht werden, bis entweder der maximal ausgebbare Schalldruckpegel $L_2$ bzw. $L_1$ erreicht ist oder ein DPOAE gemessen wird. Auf diese einfache Weise kann die Anwesenheit störender SOAE erkannt und ggf. kompensiert werden.

Des Weiteren kann das erfindungsgemäße Verfahren zur Bestimmung individueller Pegelkarten mit der unten beschriebenen Pegelkartenmethode kombiniert werden.

Zur effektiven Unterdrückung der Primärtöne $\{f_1, L_1\}$ und $\{f_2, L_2\}$ wird neben üblichen Filtermethoden vorzugsweise das *Primary-tone-phase-variation*-Verfahren von Whitehead et al., 1996, a.a.O., eingesetzt. Allgemein ist es bevorzugt, wenn zu Beginn der Messungen überprüft wird, ob die Frequenz einer der DPOAE ($f_{dp}$) mit einer spontanen Emission (SOAE) interferiert. Hier ist von Vorteil, dass Artefakte und Störquellen schon zu Beginn der Messung erkannt werden. Ist dies der Fall, kann das Verfahren so angepasst werden, dass die Abklingzeit des DPOAE so weit verlängert wird, dass sein Pegel unter eine gewisse Schwelle gelangt, bevor das nächste Primärtonpaar präsentiert wird, oder die Frequenz $f_2$ wird verschoben, um einen Mindestabstand zu der SOAE herzustellen.

Die erfindungsgemäßen Verfahren können zur Feststellung der Verstärkungsleistung des cochleären Verstärkers in einem menschlichen oder tierischen Hörorgan und vorteilhafterweise auch zur Ursache eines möglichen Funktionsverlusts herangezogen werden. Die erfindungsgemäßen Verfahren bieten sich weiterhin zur Einstellung eines Hörgeräts an.

## Figuren

Weitere Vorteile ergeben sich aus der folgenden Beschreibung der beigefügten Figuren.

**Figur 1** Vergleich der Amplituden der Einhüllenden der $2f_1$-$f_2$-DPOAE nach Anregung mittels PTA-I (graue Linie) und PTA-II (schwarze Linie).

**Figur 2** Transduktionskurve der ÄHZ und ihre Auswirkung auf das Rezeptorpotential für unterschiedlich starke DPOAE-Anregungstöne.

**Figur 3** Messung der SPDPOAE bzw. der Ruhepotentialverschiebung mit einer Anregung mit verlängertem $f_1$-Puls (Pulslänge 20 ms).

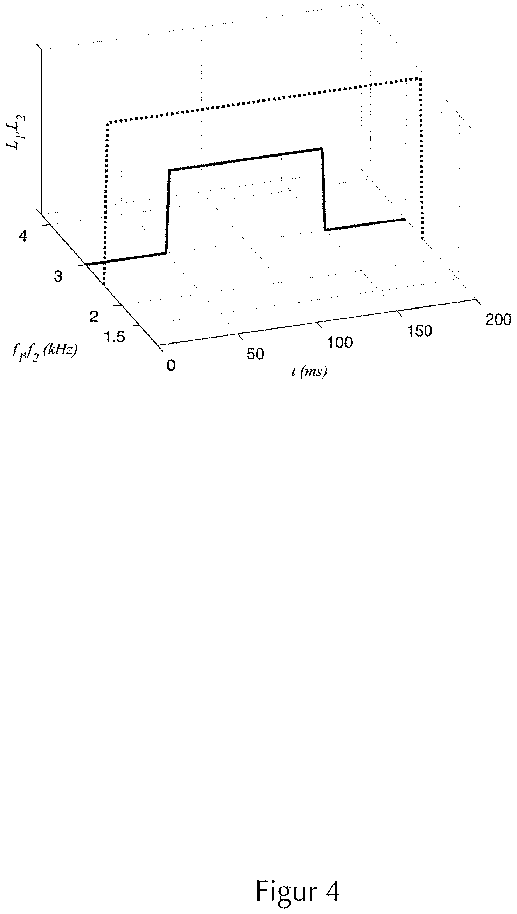

**Figur 4** Aus dem Stand der Technik bekanntes PTA-I Anregungsschema, bei dem der $f_2$-Puls (schwarz, durchgezogene Linie) während des anhaltenden $f_1$-Pulses (gestrichelte Linie) präsentiert wird.

**Figur 5** Ein System zur automatischen Bestimmung einer individuellen Funktion einer DPOAE-Pegelkarte.

**Figur 6** Eine Modellfunktion deren dreidimensionaler Graph einer Modellpegelkarte entspricht.

**Figur 7** Verfahrensschritte zur automatischen Bestimmung einer individuellen Funktion einer DPOAE-Pegelkarte.

**Figur 8** Detaillierung des verfahrensgemäßen Schrittes nach der Markierung IV in Figur 7.

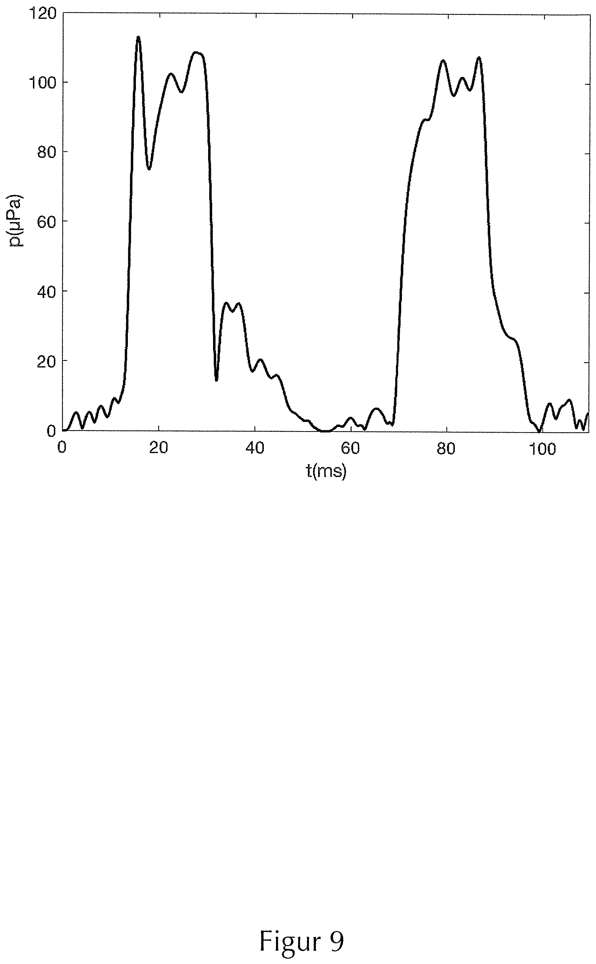

**Figur 9** Vergleich der Amplituden der Einhüllenden der $2f_1$-$f_2$-DPOAE nach Anregung mittels PTA-II (links) und PTA-I (rechts).

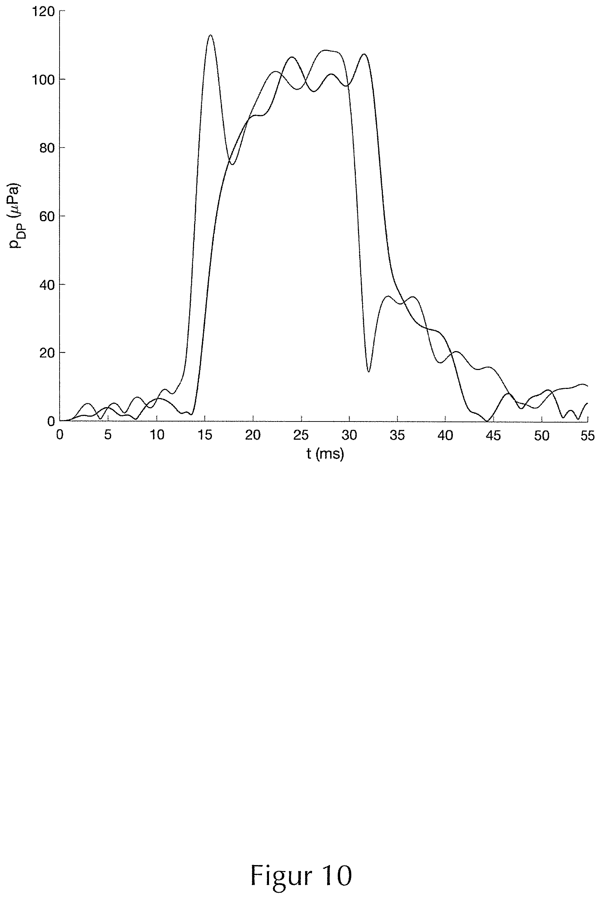

**Figur 10** Übereinandergelegte Kurven aus Figur 9.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung werden unter Bezug auf die beigefügten Zeichnungen in der nachfolgenden Beschreibung näher erläutert.

**Figur 1** zeigt die Einhüllenden der $2f_1$-$f_2$-DPOAE nach Anregung mittels PTA-I (graue Linie) und PTA-II (schwarze Linie). PTA-I und PTA-II führen zu unterschiedlichen Amplituden. Wird der $f_1$-Ton später angeschaltet als der $f_2$-Ton (hier: 5 ms später) (PTA-II), erhält man eine höhere Amplitude der Einhüllenden der $2f_1$-$f_2$-DPOAE (schwarze Linie), als wenn man umgekehrt den $f_2$-Ton um 5 ms später anschaltet als den $f_1$-Ton (graue Linie) (PTA-I).

In dem vorliegenden Beispiel zeigen die Antwortkurven des DPOAE nur die Antwort der nichtlinearen Quelle, während die Antwort der kohärenten Reflexionsquelle in diesem Probanden und bei dieser Frequenz zu gering ausfällt, um erkannt zu werden. Die Antwort der nichtlinearen Quelle ist relevant, da ihre Amplitude im Normalfall größer ist, und sie im Gegensatz zur kohärenten Reflexionsquelle von keinem zusätzlichen Prozess (Rauigkeit der Impedanzfunktion) abhängig ist.

In diesem Beispiel ist die Antwort der nichtlinearen Quelle (d.h., der Einhüllenden des $2f_1$-$f_2$-DPOAE-Zeitsignals) 3 dB größer für PTA-II als für PTA-I. Dieser Unterschied ist ausreichend groß, um sicher festgestellt werden zu können.

**Figur 2** zeigt die Transduktionskurve der ÄHZ (1) und ihre Auswirkung auf das Rezeptorpotential für unterschiedlich starke DPOAE-Anregungstöne. Input ist die Auslenkung der Stereozilien, Ouput das Rezeptorpotential, welches die Krafteinkopplung der Zelle in die Schwingungen des Organs und damit den Verstärkungsprozess steuert. Da die Ionenkanäle nur ganz geschlossen bzw. ganz geöffnet werden können, erreicht die Transduktionskurve bei hohen Eingangswerten jeweils einen Maximal- bzw. einen Minimalwert, in der hier gewählten Normierung den Wert 1 bzw. 0. Weiterhin sind zwei Eingangssignale ((2a) und (2b)) dargestellt, die einer Anregung mit zwei Primärtönen entspricht. Die resultierende Schwebung ist gut zu erkennen. Es wird einmal mit einer relativ geringen Amplitude angeregt ((2a), durchgezogene Linie), die einen noch einigermaßen linearen Teil der Transduktionskurve anspricht. Zweitens ist eine Anregung mit deutlich höherer Amplitude dargestellt ((2b), gestrichelte Linie), die eindeutig nichtlinear verarbeitet wird. Die resultierenden Ausgangssignale sind ebenfalls dargestellt. Für den Fall der Kleinsignalanregung ((3a) durchgezogene, starke Linie) ist die Systemantwort vom Eingangssignal visuell nicht zu unterscheiden. (4) stellt den Mittelwert, also den DC-Anteil des Signals dar. Bei stärkerer Anregung (5) erkennt man deutlich den Einfluss der Nichtlinearität. Aufgrund der Asymmetrie der Transduktionskurve werden die negativen Halbwellen der Potentialschwankungen stark abgeschnitten, während die positiven Halbwellen noch einigermaßen sinusförmigen Verlauf zeigen. Als Resultat verschiebt sich der Mittelwert, also der DC-Wert der Potentialschwankungen.

Damit verlagert sich im gesunden Fall der Arbeitspunkt von der steilsten Stelle hin zu einer weniger steilen. Dies muss zu einer gewissen Verringerung der Verstärkung der ÄHZ führen. Vorzugsweise erfolgt die Verschiebung des Ruhe- bzw. DC-Potentials weitgehend frequenzunabhängig innerhalb von 1-2 ms.

**Figur 3** zeigt die Messung der SPDPOAE bzw. der Ruhepotentialverschiebung mit einer Anregung mit verlängertem $f_1$-Kurzpuls. Wird der zweite Primärton {$f_2$, $L_2$} zuerst, und der verlängerte $f_1$-Kurzpuls um 5 ms verzögert angeschaltet, so erreicht im diesem Beispiel die DPOAE zu Beginn ein Maximum bei 113 μPa, fällt dann innerhalb 2 ms um mehr als 3 dB ab, und steigt dann wieder an. Dieses nachfolgende Ansteigen kann prinzipiell durch den Einfluss der zweiten Quelle entstehen, aber auch durch langsamere Regelungsprozesse der Differenz zwischen intra- und extrazellulären Potentialen. (1) Anregung mittels PTA-II, (2) Anregung mittels PTA-I.

## Pulsverschränkungsverfahren

Das erfindungsgemäße Verfahren kann außerdem mit dem aus WO 2015/192969 A1 bekannten Pulsverschränkungsverfahren ("zeitverschränkte Multifrequenzmethode") kombiniert werden.

Gepulste DPOAE haben gegenüber kontinuierlichen DPOAE den Nachteil, dass die Messung bei einer Frequenz-Schalldruckpegelkombination generell eine geringe Zeitauslastung und damit ein entsprechend geringeren Rauschabstand bei derselben Messzeit aufweist. Bei dem in WO 2015/192969 A1 beschriebenen Verfahren wird dieser Nachteil aber erheblich reduziert, indem mehrere Messungen im Zeit-Frequenzraum miteinander verschränkt werden, d.h. zeitversetzt abwechselnd präsentiert werden. So können innerhalb eines Blocks zeitversetzt bspw. sieben Frequenzen stimuliert und analysiert werden. Das Messverfahren wird also durch parallele und adaptive Stimulations- und Analyseschritte beschleunigt, und erlaubt die Messung von Wachstumskurven für 5-7 Frequenzen $f_2$ in typischerweise 2-2,5 min.

Bei dem in WO 2015/192969 A1 beschriebenen Verfahren werden zumindest zwei unterschiedliche Pulspaare (davon jedes mit einem gepulsten ersten Primärton $\{f_1, L_1\}$ und einem gepulsten zweiten Primärton $\{f_1, L_1\}$) mit unterschiedlichen Anregungsfrequenzen $f_2$ (und folglich auch unterschiedlichen Anregungsfrequenzen $f_1$) in einem Block präsentiert, der während einer Messdauer mehrfach wiederholt wird. In einem Block folgen also auf ein erstes gepulst präsentiertes Primärtonpaar mit $\{f_{2,1}, f_{1,1}\}$ ein zweites Primärtonpaar mit unterschiedlichen Frequenzen $\{f_{2,2}, f_{1,2}\}$ und ggf. weitere mit $\{f_{2,n}, f_{1,n}\}$, wobei das Frequenzverhältnis vorzugsweise immer nahe bei $f_{2,n}/f_{1,n}$ = 1,2 gehalten wird.

WO 2015/192969 A1 sieht vor, dass die gepulsten Primärtöne eines jeden Paares nach dem PTA-I Anregungsschema (mit $f_2$-Kurzpuls) präsentiert werden. Dabei setzt der $f_1$-Puls eines Pulspaares vor dem $f_2$-Puls ein, und endet nachdem der $f_2$-Puls beendet wurde, d.h. der $f_1$-Puls ist länger als $f_2$-Puls eines Pulspaares.

Das erfindungsgemäße Pulsverschränkungsverfahren sieht die Präsentation von zumindest einem Primärtonpaar nach dem PTA-II Anregungsschema (mit $f_1$-Kurzpuls) vor. Die weiteren Primärtonpaare in einem Block können entweder gemäß PTA-II (reines PTA-II Pulsverschränkungsverfahren) oder gemäß PTA-I (kombiniertes PTA-I/PTA-II Pulsverschränkungsverfahren) präsentiert werden.

Im erfindungsgemäßen Pulsverschränkungsverfahren folgt in einem Block der Beginn eines (PTA-I oder PTA-II-) Pulspaares vorzugsweise mit einem zeitlichen Abstand T ($T_{SLOT}$ oder $T_S$) auf den Beginn des im Block unmittelbar vorhergehenden (PTA-I oder PTA-II-)Pulspaares, wobei $T_S$ in der Regel mindestens der Länge des vorausgegangenen Pulses entspricht. Vorzugsweise ist $T_S$ > 10 ms. Diese für ein (PTA-I- oder PTA-II-)Pulspaar in einem Block reservierte Messzeit wird nachfolgend auch als Slot bezeichnet. Es ist dabei zu bemerken, dass Slots sich nicht überlappen, sondern ein Slot dem anderen folgt, wenn der vorhergehende Slot beendet ist.

Vorteilhafterweise wird auf diese Weise das zweite (PTA-I oder PTA-II-)Pulspaar erst präsentiert, wenn die durch das erste (PTA-I oder PTA-II-)Pulspaar evozierte DPOAE hinreichend (auf ca. 1 bis 10% des Ausgangswertes) abgeklungen ist, so dass es zu keinen merklichen Störeinflüssen bei der Messung der Schalldruckpegel ($L_{dp}$) der einzelnen DPOAE kommt, die verglichen mit den Schalldruckpegeln $L_1$ und $L_2$ der $f_1$- und $f_2$-Pulse nur einen sehr geringen Schalldruckpegel aufweisen. Zudem ermöglicht der erhöhte zeitliche Abstand zwischen der Präsentation von (PTA-I oder PTA-II-)Pulspaaren mit gleichen Anregungsfrequenzen $f_1$ und $f_2$ eine hinreichende Erholungszeit zum vollständigen Abklingen des im vorhergehenden Messblock ausgelösten DPOAE.

Vorzugsweise kann der oder jeder Block von (PTA-I oder PTA-II-)Pulspaaren während einer Blockzeit $T_B$ präsentiert werden. Die Blockzeit $T_B$ ist definiert als die Summe der Slotlängen $T_S$ eines Blocks. Vorzugsweise wird $T_B$ vorzugsweise so gewählt, dass ein (PTA-I oder PTA-II-)Puls ausreichend angeklungen ist, wenn er wiederholt wird. Vorzugsweise liegt zwischen dem Beginn eines ersten und eines folgenden Pulspaares mit derselben Anregungsfrequenz $f_2$ ein zeitlicher Abstand von 30 ms bis 100 ms, vorzugsweise von zumindest 70 ms.

Vorzugsweise liegen die $f_2$-Anregungsfrequenzen von zwei unmittelbar aufeinander folgenden (PTA-I oder PTA-II-)Pulspaaren in einem Block mindestens eine Oktave auseinander. Die Wahl eines derartigen Frequenzabstandes von mindestens eines Oktave zwischen den $f_2$-Anregungsfrequenzen sorgt vorteilhafterweise dafür, dass auch die Frequenzen $f_{dp}$ der jeweils evozierten DPOAE hinreichend groß auseinander liegen, sodass es vorzugsweise zu keinen merklichen Störeinflüssen bei der Messung der einzelnen DPOAE kommt.

Für die Anregungsfrequenzen $f_1$ und $f_2$ gilt weiterhin das oben gesagte, beispielsweise ein bevorzugtes Frequenzverhätnis von $f_1/f_2$=1,2. Ein Beispiel für zwei unterschiedliche Pulspaare eines Blocks sind ein erstes Pulspaar mit einer Anregungsfrequenz $f_2$ von 1,5 kHz und einer Anregungsfrequenz $f_1$ von 1,25 kHz und ein zweites Pulspaare mit einer Anregungsfrequenz $f_2$ von 4 kHz und einer Anregungsfrequenz $f_1$ von 3,33 kHz.

Ein bevorzugter Satz (d.h. Panel oder Frequenzzeitmuster der Anregungsfrequenzen in einem Block.) von $f_2$-Anregungsfrequenzen in einem Block besteht aus den Anregungsfrequenzen $f_2$ = 1 kHz, $f_2$ = 3 kHz, $f_2$ = 1,5 kHz, $f_2$ = 6 kHz. Diese $f_2$-Anregungsfrequenzen werden in einem Block in dieser Reihenfolge wiederholt präsentiert. Ein anderer bevorzugter Satz (Panel) von $f_2$-Anregungsfrequenzen in einem Block besteht aus den Anregungsfrequenzen $f_2$ = 2 kHz, $f_2$ = 4 kHz, $f_2$ = 1,5 kHz, $f_2$ = 3 kHz.

Der (PTA-I) $f_1$-Puls kann frequenzabhängig vorzugsweise 3-10 ms früher ein- und 3-10 ms später ausgeschaltet als der (PTA-I) $f_2$-Puls, so dass der (PTA-I) $f_1$-Puls während der Präsentation des (PTA-I) $f_2$-Pulses kurzzeitig einen Gleichgewichtszustand erreicht.

Es kann aber auch zur größtmöglichen Zeitersparnis mit zwei gleich oder ähnlich kurzen (PTA-I) $f_1$ und $f_2$-Pulsen gearbeitet werden, die so zeitversetzt werden, dass am diagnostisch wertvollsten Abbildungsort des (PTA-I) $f_2$-Pulses in

der Cochlea beide Anregungen gleichzeitig stattfinden. Dann wird der (PTA-I) $f_1$-Puls etwa 0.1-3 ms später angeschaltet, da seine Laufzeit zum näher basal (Richtung Fußplatte) gelegenen Abbildungsort des $f_2$-Pulses kürzer ist als für den (PTA-I) $f_2$-Puls. Ist diese Einstellung optimal gewählt, tritt noch kein Effekt durch die afferent-efferente Rückkopplungsschleife des medialen olivocochleären Reflexes ein.

Die Dauer (Länge) des (PTA-I) $f_1$ und $f_2$-Pulses in einem Pulspaar kann vorzugsweise 2 bis 20 ms. In Bezug auf die Pulslänge gilt ansonsten das oben in Bezug auf PTA-II gesagte.

Im Pulsverschränkungsverfahren der vorliegenden Erfindung kann die Abfolge der Pulspaare und der zeitliche Abstand zwischen zwei aufeinanderfolgenden (PTA-I und/oder PTA-II) Pulspaaren, also die Slotzeit ($T_S$), in einem Block konstant sein. Bei dieser blockstarren Vorgehensweise werden so viele Blöcke gemessen und gemittelt, bis für jede Anregungsfrequenz in dem Satz (Panel) das gewünschte SNR erreicht ist. Alternativ können aber auch bei Erreichen des gewünschten SNR für eine Anregungsfrequenz $f_2$ die noch vorgesehenen Pulspaare für diese Anregungsfrequenz $f_2$ und folglich deren Mittelungen übersprungen werden. Dazu kann mit den noch verbleibenden Pulspaaren in verkürzten Blöcken, also mit weniger Slots, weiter gemessen werden, was die Messzeit weiter verkürzt.

Weiterhin können im erfindungsgemäßen Pulsverschränkungsverfahren der vorliegenden Erfindung zwei Sätze mit zumindest teilweise hinsichtlich der zweiten Anregungsfrequenz $f_2$ unterschiedlichen (PTA-I und/oder PTA-II) Pulspaaren ausgewählt werden, wobei die Blöcke der einzelnen Sätze zeitlich nacheinander präsentiert und die DPOAE gemessen und gemittelt werden. Die Sätze werden also nacheinander abgearbeitet. In diesem blockflexiblen Verfahren mit fester Pulsanordnung werden beispielsweise sieben (PTA-I und/oder PTA-II) Pulspaare mit unterschiedlichen Anregungsfrequenzen $f_2$ so angeordnet, das sie nach allgemeiner Erfahrung alle etwa die Mittelungszeit zugeteilt bekommen, die benötigt wird, um einen bestimmten Rauschabstand zu erzielen.

Vorzugsweise werden erfindungsgemäß mehrere Sätze nacheinander präsentiert, auf die (PTA-I und/oder PTA-II) Pulspaare so verteilt werden, dass (PTA-I und/oder PTA-II) Pulspaare mit niedrigen Anregungsfrequenzen $f_2$ (und damit auch niedrigen Anregungsfrequenzen $f_1$) in mehreren Sätzen vorkommen.

Im erfindungsgemäßen Pulsverschränkungsverfahren kann weiterhin für jedes (PTA-I und/oder PTA-II) Pulspaar laufend überprüft werden, ob ein gewünschtes SNR erreicht wird. Bei der weiteren Messung die (PTA-I und/oder PTA-II) Pulspaare kann für diese Anregungsfrequenz $f_2$ eliminiert und die verbleibenden (PTA-I und/oder PTA-II) Pulspaare ggf. neu auf die Blöcke verteilt werden.

Bei diesem blockflexiblen Verfahren mit freier (PTA-I und/oder PTA-II) Pulspaaranordnung wird die Länge der Messungen für die einzelnen (PTA-I und/oder PTA-II) Pulspaare nicht mehr relativ zueinander festgeschrieben. Stattdessen wird für jedes (PTA-I und/oder PTA-II) Pulspaar laufend überprüft, ob das SNR erreicht ist; sobald das der Fall ist, wird geprüft, ob noch ein weiteres (PTA-I und/oder PTA-II) Pulspaar unvollendet ist. Auf diese Weise werden die fertig gemessenen (PTA-I und/oder PTA-II) Pulspaare nacheinander aus der Messung eliminiert, und nur die verbleibenden (PTA-I und/oder PTA-II) Pulspaare weiter präsentiert. Dabei wird einerseits überprüft, ob der Oktavabstand zwischen zwei aufeinander folgenden (PTA-I und/oder PTA-II) Pulspaaren eingehalten wird. Ist dies nicht mehr der Fall, werden (PTA-I/PTA-II)Pulspaare u.U. nicht mehr in den Blöcken abgearbeitet, denen sie ursprünglich zugeordnet wurden, sondern in anderen (neu definierten) Blöcken. Zudem wird überprüft, ob der erforderliche zeitliche Abstand $T$ (entsprechend der Zeit für einen Slot ($T_S$) zzgl. der notwendigen Abklingzeit ($T_{Abkl}$)) zwischen (PTA-I und/oder PTA-II) Pulspaaren mit gleicher Anregungsfrequenz $f_2$ eingehalten wird.

Wie oben Hinblick auf PTA-II beschrieben, werden auch innerhalb eines Blocks die Schalldruckpegel der (PTA-I und/oder PTA-II)Pulspaare vorzugsweise ähnlich gewählt.

Vorzugsweise wird die DPOAE für alle in dem oder den Sätzen enthaltenen Anregungsfrequenzen $f_2$ bei einem jeweils der Anregungsfrequenz zugeordneten Schalldruckpegel $L_2$ gemessen und gemittelt, und dann zumindest eine erneute Messung bei neuen Schalldruckpegeln $L_2$ durchgeführt, wobei bevorzugt in einem Schwellwertannäherungsverfahren aus den gemessenen DPOAE für jede Anregungsfrequenz $f_2$ der neue Schalldruckpegel $L_2$ für die jeweils erneute Messung bestimmt wird. Dieses Verfahren wird wiederholt, bis für jede Anregungsfrequenz $f_2$ eine Wachstumskurve aus Messwerten der Schalldruckpegel der DPOAE für 3 bis 4 unterschiedlichen Schalldruckpegel $L_2$ bestimmt werden kann, aus der dann die jeweiligen Schwellwerte bestimmt werden. Eine genauere Beschreibung dieses Verfahrens findet sich in WO 2015/192969 A1.

## Pegelkartenmethode

Das erfindungsgemäße Verfahren kann mit der aus PCT/EP2017/000334 bekannten Pegelkartenmethode kombiniert werden. Eine "DPOAE-Pegelkarte" (DPOAE level map, vgl. Shera und Guinan, J Acoust Soc Am. 2007 Feb;121(2):1003-16) und Martin et al. J. Acoust. Soc. Am. 127 5 , p. 2955-2972) bezeichnet dabei die Amplitude eines Pegels (hier des *2f1-f2*-Distorsionsprodukts) in Abhängigkeit von den Primärtonpegeln.

Das in PCT/EP/2017/000334 beschriebene Verfahren dient der automatischen Bestimmung einer individuellen Funktion einer DPOAE-Pegelkarte. Dabei werden vorzugsweise Fehler bei der Extrapolation der Wachstumsfunktionen vermieden, die in andere im Stand der Technik bekannten Verfahren zur Messung der Distorsionsproduktschwelle $L_{edpt}$

prinzipbedingt vorkommen können. Das in PCT/EP2017/000334 beschriebene Verfahren kann für konventionelle (also quasi kontinuierlich) gemessene (PTA-II angeregte) DPOAE eingesetzt werden. Es kann aber auch mit einem Verfahren der gepulsten (PTA-II angeregten) DPOAE kombiniert werden, wie beschrieben in DE 102014108663 A1. Insbesondere kann das in PCT/EP2017/000334 beschriebene Verfahren mit der oben beschriebenen kombinierten Pulsverschränkung kombiniert werden.

Insbesondere können die Verfahren der vorliegenden Erfindung die folgenden Schritte zur automatischen Bestimmung einer individuellen Funktion einer DPOAE-Pegelkarte mit $p_{dp,I} = f(L_1, L_2)$ eines menschlichen oder tierischen Gehörs enthalten:

- Einlesen einer Modellfunktion $p_{dp,M} = f(L_1, L_2)$ mit Modellparametern einer DPOAE-Pegelkarte, basierend auf einer Anzahl von $N$ DPOAE-Messungen eines Anregungsfrequenzpaars $\{f_1, f_2\}$ mit jeweils unterschiedlichen Pegelpaaren $\{L_1^{(1...N)}, L_2^{(1...N)}\}$ bei einer Population ($p$) von Normalhörenden, in einen Arbeitsspeicher einer Rechnereinheit, wobei $N \geq 40$ und $p \geq 2$ ist,

- automatische Präsentation von n unterschiedlichen Pegelpaaren $\{L_1^{(1...n)}, L_2^{(1...n)}\}$ eines Anregungsfrequenzpaars $\{f_1, f_2\}$ über Tonausgabemittel an ein Individuum und Erfassen der korrespondierenden DPOAE des Individuums über Tonaufnahmemittel, wobei wenigstens das erste Pegelpaar $\{L_1^{(1)}, L_2^{(1)}\}$ vordefiniert ist und wobei n $\ll$ $N$ ist,

- iteratives Anpassen der Modellfunktion $p_{dp,M} = f(L_1, L_2)$ an die gemessenen n DPOAE bis zum Erhalt einer individuellen Funktion $p_{dp,I} = f(L_1, L_2)$ mit individuellen Parametern einer DPOAE-Pegelkarte des Individuums durch die Rechnereinheit, und

- Ausgabe der individuellen Funktion $p_{dp,I} = f(L_1, L_2)$ und/oder deren individueller Parameter an einer Ausgabeeinheit der Rechnereinheit.

Bei der iterativen Anpassung (*curve fitting,* Kurvenanpassung) wird die Modellfunktion an experimentell ermittelte Messwerte angepasst. Dazu werden Parameter dieser Funktion mit einem geeigneten Algorithmus so lange geändert, bis die Abweichung zwischen den Messwerten und der schrittweise veränderten Funktion entsprechend einem Optimalitätskriterium minimal ist (z.B. Minimierung des quadratischen Fehlers). Algorithmen zu einer derartigen iterativen Anpassung sind dem Fachmann aus dem Stand der Technik bekannt (z.B. lsqcurvefit oder lsqnonlin).

Das erste Pegelpaar $\{L_1^{(1)}, L_2^{(1)}\}$ kann einen Pegel $L_1$ von 67 $\pm$ 10 dB und einen Pegel $L_2$ von 57 $\pm$ 10 dB aufweisen. Diese Pegel von L$_1$ und von L$_2$ haben sich als besonders günstige Anfangspegel erwiesen. Bei Normalhörenden befinden sich diese Anregungspegel nämlich noch in dem Bereich, bis zu welchem die Pegelkarte näherungsweise linear ansteigt, und selbst bei Hörverlusten bis ca. 40 dB kann immer noch ein DPOAE bei diesen Pegeln gemessen werden. Man erhält also in den meisten Fällen Werte, die für die Erfassung der Pegelkarte valide sind.

Die Modellfunktion definiert einen linear ansteigenden Grat, dem linear miteinander verknüpfte $\{L_1^{(G)}, L_2^{(G)}\}$ Pegelpaare zugeordnet sind (wobei "G" der Index für "dem Grat zugeordnet" ist). Vorzugsweise können mindestens die Hälfte der gemessenen Pegelpaare $\{L_1^{(i)}, L_2^{(i)}\}$ um mindestens 5 dB zu beiden Seiten abseits der dem Grat zugeordneten $\{L_1^{(G)}, L_2^{(G)}\}$ Pegelpaare liegen (wobei "i" der Index der Messung von 1 bis n ist).

Vorzugsweise werden die unterschiedlichen Pegelpaare $\{L_1^{(i)}, L_2^{(i)}\}$ in einer Sequenz präsentiert, die für jedes Individuum identisch ist. Durch dieses stark vereinfachte und vereinheitlichte (starre) Verfahren wird zwar die Annäherung an die individuelle Funktion einer Pegelkarte etwas weniger genau, jedoch ist dieses Verfahren in der Durchführung sehr schnell.

Von Vorteil kann es ferner sein, wenn die vordefinierten, unterschiedlichen Pegelpaare $\{L_1, L_2\}$ in einer Sequenz präsentiert werden, die eine Anzahl von $k$ Subsequenzen aufweist, deren Pegelpaare $\{L_1, L_2\}$ im Wesentlichen quer zu den linear miteinander verknüpften und dem Grat zugeordneten Pegelpaaren $\{L_1^{(G)}, L_2^{(G)}\}$ liegen. Durch die Einschaltung der Subsequenzen kann der Grat an mehreren Stellen abgetastet werden, was die Genauigkeit der Bestimmung der individuellen Funktion der DPOAE-Pegelkarte erhöht.

Vorzugweise ist $n \geq 5$ und $\leq 12$, vorzugsweise $6 \leq n \leq 8$. Durch die kleine Anzahl an vorgesehenen Messungen wird eine kurze Messdauer erzielt bei gleichzeitig guter Erfassung der individuellen Funktion der DPOAE-Pegelkarte.

Vorteilhaft ist die Anzahl der Subsequenzen $k \geq 2$ und $\leq 5$, wodurch eine gute Abtastung des Grats der DPOAE Pegelkarte erreicht wird.

Weiter vorteilhaft ist es, wenn die einem ersten vordefinierten Pegelpaar $\{L_1^{(1)}\ L_2^{(1)}\}$ nachfolgenden Pegelpaare $\{L_1^{(2...n_k)},\ L_2^{(2...n_k)}\}$ einer Subsequenz mit $n_k$ Messungen über eine Funktion

$$\{L_1^{(i)},\ L_2^{(i)}\} = \{L_1^{(i-1)} + \mu \cdot \Delta L_1,\ L_2^{(i-1)} + \mu \cdot \Delta L_2\}$$ aus dem jeweils vorhergehenden Pegelpaar

$L_1^{(i-1)},\ L_2^{(i-1)}$ bestimmt werden, wobei $\mu = \pm 1$, insbesondere +1 ist, und $\Delta L_1$, $\Delta L_2$ ein Pegelabstand von zwei aufeinanderfolgenden Pegelpaaren ist und Werte von $\Delta L_1 = 4$ bis 14 dB, vorzugsweise von 6 bis 10 dB, und $\Delta L_2 = 0$ bis - 2,78 dB, vorzugsweise $\Delta L_2 = - 1,52$ bis - 2,78 dB aufweist. Der Faktor $\mu$ legt dabei die Suchrichtung (zu kleineren oder größeren $L_1$-Meßpegeln hin) quer zum Grat fest.

Vorzugsweise wird, wenn das erste Pegelpaar $\{L_1^{(1)}, L_2^{(1)}\}$ und das zweite Pegelpaar $\{L_1^{(2)}, L_2^{(2)}\}$ zwei DPOAE mit $p_{dp,l}^{(1...2)}$ produziert, die einen Rauschabstand von je >=4 dB, vorzugsweise >=10 dB aufweisen, der Pegel eines nachfolgenden dritten Pegelpaars $\{L_1^{(3)},\ L_2^{(3)}\}$ wenigstens um $\Delta L_1 \geq 4$ dB unterschiedlich eingestellt als der Pegel des vorhergehenden Pegelpaars $\{L_1^{(2)}, L_2^{(2)}\}$, wenn $p_{dp,l}^{(2)} - p_{dp,l}^{(1)} > 0$, und wird andererseits der Pegel eines nachfolgendes Pegelpaars $\{L_1^{(3)},\ L_2^{(3)}\}$ wenigstens um $\Delta L_1 \leq - 4$ dB unterschiedlich eingestellt als der Pegel des ersten Pegelpaars $\{L_1^{(1)}, L_2^{(1)}\}$, wenn $p_{dp,l}^{(2)} - p_{dp,l}^{(1)} \leq 0$. Mit dieser Vorgehensweise wird erreicht, dass wenigstens ein Punkt links und ein Punkt rechts des Grates und dazwischenliegend ein Punkt in der Nähe des Grates gemessen wird.

Vorzugsweise wird, wenn das erste Pegelpaar $\{L_1^{(1)},\ L_2^{(1)}\}$ keine DPOAE mit $p_{dp,l}^{(1)}$ produziert, die einen Rauschabstand von >=4 dB, vorzugsweise >=10 dB aufweisen, in derselben Suchrichtung weiterverfahren, bis entweder der maximale bzw. minimale Anregungspegel $L_1^{(i)}$ erreicht ist, oder eine Gruppe von drei validen DPOAE mit $p_{dp,l}^{(i..i+2)}$ produziert wurde, die einen Rauschabstand von je >=4 dB, vorzugsweise >=10 dB aufweisen. Hiermit wird gegenüber einem starren Verfahren erreicht, dass der Grat auch dann aufgefunden wird, wenn er deutlich neben der für Normalhörende zu erwartenden Position liegt, wie es etwa bei Schallleitungsstörungen der Fall sein kann.

Vorzugsweise wird, wenn in der ersten Subsequenz nach Messung bei *i* Anregungspegelpaaren keine Gruppe von drei validen DPOAE produziert wird, die einen Rauschabstand von je >=4 dB, vorzugsweise >=10 dB aufweisen, eine weitere Subsequenz mit höherem Pegelpaar $\{L_1^{(i+1)}, L_2^{(i+1)}\}$ gestartet, wobei das Startpegelpaar für die erneute Subsequenz auf $L_2^{(i+3)} = L_2^{(1)} + 20 \pm 10\ dB$, $L_1^{(i+3)} = L_1^{(1)} + 20 \pm 10\ dB$ eingestellt wird. Der Pegel wird vorzugsweise auf den technisch maximal erreichbaren bzw. sinnvollen Pegel begrenzt. Dieser maximale Pegel kann z.B. bei 75-85 dB SPL Schalldruck liegen. Durch diese Vorgehensweise, können auch stark vom Durchschnitt abweichende individuelle Pegelkarten bzw. deren Funktion noch ermittelt werden.

Vorzugsweise wird, nach der Erfassung der DPOAE von wenigstens 3 Pegelpaaren $\{L_1^{(1..3)},\ L_2^{(1..3)}\}$, die vorzugsweise einer Subsequenz zugeordnet sind, aus diesen 3 Pegelpaaren $\{L_1^{(1..3)},\ L_2^{(1..3)}\}$ die Lage des Grates $\{L_1^{(G)},\ L_2^{(G)}\}$ längs der durch die 3 Pegelpaare gebildeten Linie ermittelt und im Anschluss daran ein viertes Pegelpaar $\{L_1^{(4)},\ L_2^{(4)}\}$ präsentiert, welches in einem vorgegebenen Abstand gratabwärts liegt, wobei der Gruppenmittelwert der Gratrichtung, $\varphi$ verwendet wird, und wobei anhand der aus den vier präsentierten Pegelpaaren $\{L_1^{(1..4)},\ L_2^{(1..4)}\}$ bestimmten DPOAE eine Steigung m des linearen Grats der Pegelkarte ermittelt wird.

Vorzugsweise wird, wenn in der ersten oder zweiten Subsequenz eine Gruppe von drei validen DPOAE mit $p_{dp,l}^{(i-2...i)}$ produziert wird, die einen Rauschabstand von je >=4 dB, vorzugsweise >=10 dB aufweisen, durch automatische Anpassung einer geeigneten Berechnungsfunktion an die zugehörigen DPOAE $p_{dp,l}^{(i...i-2)}$ das Pegelpaar unterhalb des Grates $\{L_1^{(G)},\ L_2^{(G)}\} = \{L_1^{(i-2)} + \varepsilon \cdot \Delta L_1, L_2^{(i-2)} + \varepsilon \cdot \Delta L_2\}$ bestimmt, wobei $\varepsilon$ so berechnet wird, daß

$p_{dp,I}\left(L_1^{(G)},\ L_2^{(G)}\right)$ ein Maximum bildet, und davon ausgehend ein viertes Pegelpaar $\{L_1^{(i+1)},\ L_2^{(i+1)}\}$ präsentiert wird, mit einer Funktion $\{L_1^{(i+1)},\ L_2^{(i+1)}\} = \{L_1^{(i)} +\ \Delta L_1, L_2^{(i)} +\ \Delta L_2\}$, wobei $\Delta L_2 = -15 \pm 10\ dB$ eingestellt wird, und das Pegelpaar vorzugsweise auf der Projektion des erwarteten Grates auf die L1,L2-Ebene, d.h. mit $\Delta L_1/\Delta L_2 \approx 0.51 \pm 0.15$ eingestellt wird, und wobei anhand der aus den vier präsentierten Pegelpaaren $L_1^{(i-2\dots i+1)}$, $L_2^{(i-2\dots i+1)}$ bestimmten DPOAE die Steigung m des näherungsweise linearen Grats der Pegelkarte ermittelt wird.

Anhand der ermittelten Steigung m des linearen Grats der Pegelkarte können z.B. wenigstens zwei, vorzugsweise drei weitere Pegelpaare $L_1^{(i+1\dots i+3)}$, $L_2^{(i+1\dots i+3)}$ automatisch definiert werden, deren Anregungspegel in einer Subsequenz formiert werden, und die aufgrund der bereits bekannten Lage und Steigung des Grates so bestimmt werden, dass erwartet werden kann, valide DPOAE innerhalb einer Messzeit von je $t_m \leq 40\ s$ zu messen, wozu eine Modellfunktion an die vorzugsweise vier bereits valide gemessenen DPOAE angepasst wird, und dann in der Modellfunktion die letzten zwei oder drei Pegelpaare so bestimmt werden, dass die erwarteten DPOAE-Pegel vorzugsweise bei $p_{DP\ I}^{(i+1\dots i+3)}$, $p_{DP\ I}^{(i+1\dots i+3)} \geq 10\ \mu\mathrm{Pa}$ liegen.

Vorzugsweise werden die Pegelpaare $\{L_1^{(1-n)},\ L_2^{(1-n)}\}$ gepulst präsentiert, wobei jeder einzelne Puls mit einer Dauer $T_D$ von 2 bis 40 ms präsentiert wird. Durch die Verwendung einer solchen gepulsten Präsentation kann der Einfluss der beiden Quellbeiträge einer DPOAE unterdrückt bzw. separiert werden.

Vorzugsweise werden die Pegelpaare $\{L_1^{(1.n)},\ L_2^{(1.n)}\}$ in Blöcken aus mehreren, zeitlich nacheinander in Pulsen präsentierten Pegelpaaren $\{L_1^{(1..n)},\ L_2^{(1..n)}\}$ präsentiert, wobei zeitlich direkt aufeinanderfolgende Pegelpaare $\{L_1^{(1..n)},\ L_2^{(1..n)}\}$ unterschiedliche Anregungsfrequenzen $\{f_2, f_1\}$ aufweisen. In einem Block folgen also auf ein erstes gepulst präsentiertes Pegelpaar mit $\{f_{2,1}, f_{1,1}\}$ ein zweites Pegelpaar mit unterschiedlichen Frequenzen $\{f_{2,2}, f_{1,2}\}$ und ggf. weitere mit $\{f_{2,m}, f_{1,m}\}$, wobei das Frequenzverhältnis immer nahe bei $f_{2,m}/f_{1,m} = 1,2$ gehalten wird. Mehrere Blöcke mit Zeit-Frequenz-verschränkten Pulspaaren können gemittelt werden, bevor eine Auswertung erfolgt. Durch diese Maßnahme wird es möglich, die Zeit, in der die Pulsantwort auf eine Präsentation bei einem Frequenzpaar abklingt, zu nutzen, um bei einer anderen Frequenz zu messen, und so die Messzeit gegenüber einer hinsichtlich der gewünschten Messfrequenzen rein sequentiellen Vorgehensweise zu reduzieren.

Vorteilhaft wird in einem Verfahrensschritt, die ermittelte individuelle Funktion einer DPOAE-Pegelkarte und deren Parameter von der Rechnereinheit in einem nicht-flüchtiger Speicher abgespeichert. Ebenfalls können die ermittelten Rohdaten von der Rechnereinheit in dem nicht-flüchtiger Speicher abgespeichert werden. Die abgespeicherten Daten können von der Rechnereinheit zur kontinuierlichen Erweiterung des, der Modellfunktion einer Pegelkarte zugrundeliegenden Datensatzes verwendet werden.

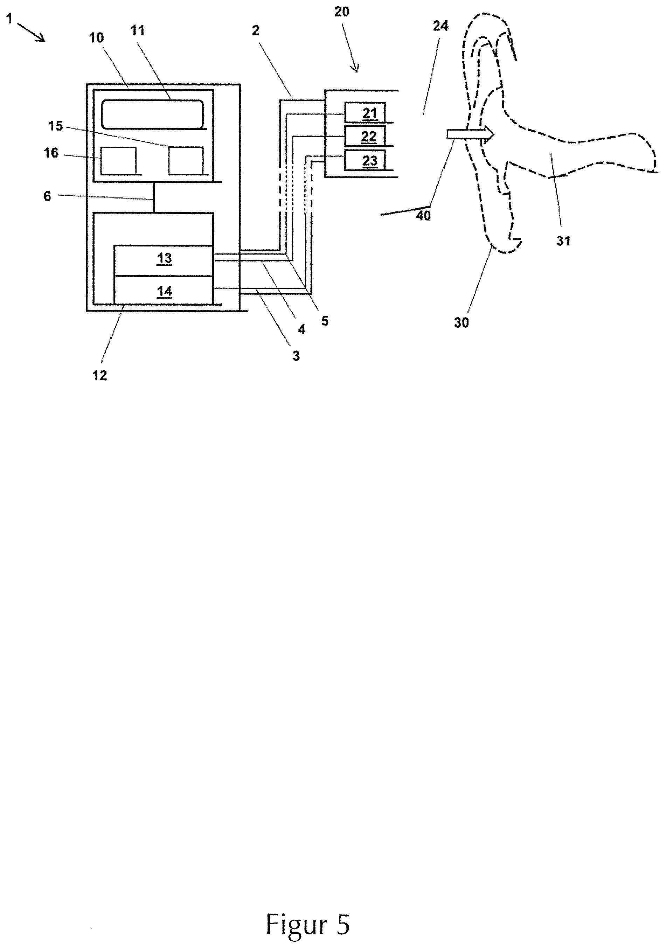

In Figur 5 ist ein System zur automatischen Bestimmung einer individuellen Funktion einer DPOAE-Pegelkarte eines menschlichen oder tierischen Gehörs in einer möglichen Ausgestaltung dargestellt. Zu dem System 1 gehören eine an einem Ohr positionierbare Sonden-Einheit 20, insbesondere eine OAE-Sonde, und eine Rechnereinheit 10. Die Sonden-Einheit weist eine Sondenspitze 24 auf, die in den Gehörgang eines Ohres einführbar ist. In der Sonden-Einheit 20 ist ein Tonaufnahmemittel 23, wie z.B. ein Mikrofon, angeordnet, welches zur Aufnahme von aus dem Gehörgang kommenden Tönen eingerichtet ist. In der Sonden-Einheit 20 sind ferner ein erstes und ein zweites Tonausgabemittel 21 und 22 vorgesehen, die als f1-Schallgeber (Tonausgabemittel 21) und als f2-Schallgeber (Tonausgabemittel 22) fungieren. Die Tonausgabemittel 21, 22 können dabei z. B. als Lautsprecher ausgebildet sein. Es kann auch nur ein Tonausgabemittel bzw. nur ein Lautsprecher vorgesehen sein, das/der zum gleichzeitigen Abspielen zweier Töne $f_1$, $f_2$ eingerichtet ist und insbesondere eine hochlineare Charakteristik besitzt. Die Sonden-Einheit 20 ist z.B. über eine Kabelverbindung 2 mit der Steuereinheit, die die Rechnereinheit 10 beinhaltet, verbunden. In der Kabelverbindung 2 sind vorzugsweise geschirmte Leitungen 3, 4, 5 vorhanden über welche die Tonausgabemittel 21, 22 sowie das Tonaufnahmemittel 23 mit einer AD/DA-Wandlereinheit 12 der Steuereinheit verbunden sind. Die AD/DA-Wandlereinheit 12 ist ihrerseits mit der Rechnereinheit 10 über wenigstens eine Leitung 6 für einen bi-direktionalen Datenaustausch verbunden. Alternativ zu der Kabelverbindung 2 könnte die Sonden-Einheit 20 auch drahtlos mit der Steuereinheit bzw. mit der Rechnereinheit 10 kommunizieren. Die drahtlose Verbindung könnte z. B. eine bluetooth-Funkstrecke oder eine andere geeignete Funkverbindung sein, die vorzugsweise eine kurze Reichweite aufweist.

Die Rechnereinheit 10 verfügt über einen Arbeitsspeicher 15 und über einen nicht-flüchtigen Speicher 16 in dem eine Modellfunktion $p_{dp,M} = f(L_1, L_2)$ für eine Modellpegelkarte eines menschlichen oder tierischen Gehörs und die zu dieser Modellfunktion gehörenden Parameter gespeichert sind. Ebenfalls ist in dem nicht-flüchtigen Speicher 16 die Anweisung zum Durchführen eines hier beschriebenen Verfahrens hinterlegt. Das System 1 verfügt ferner über ein Ausgabemittel 11 bzw. eine Anzeigeeinheit, wie z.B. ein Display, einen Monitor oder dergleichen, über die eine ermittelte einer individuellen Funktion einer DPOAE-Pegelkarte eines menschlichen oder tierischen Gehörs und deren Parameter von dem System 1 ausgegeben werden und einem Nutzer zugänglich gemacht werden können. Das Ausgabemittel 11 kann auch in Form einer Schnittstelle realisiert sein, über die ein externes Ausgabegerät, wie z.B. ein Drucker oder ein Monitor an das System angeschlossen werden können.

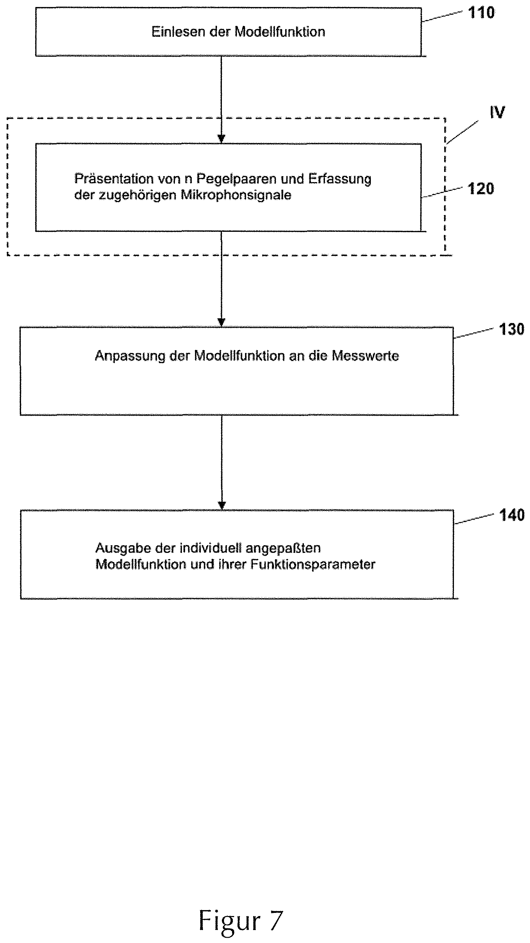

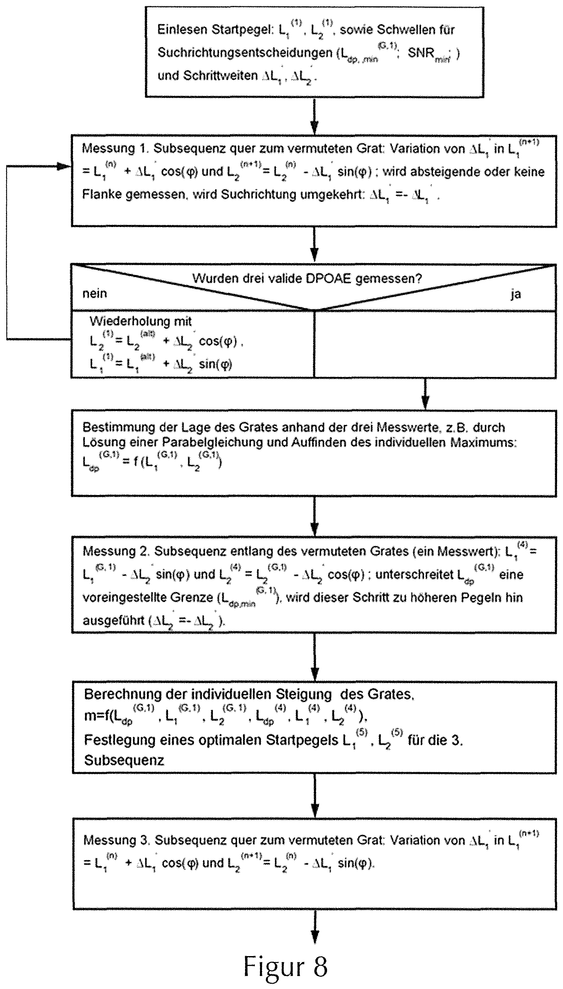

Zur Durchführung eines automatischen Messvorgangs zur Erstellung einer individuellen Funktion einer DPOAE-Pegelkarte eines menschlichen oder tierischen Gehörs wird die Sonden-Einheit 23 in Richtung des Pfeils 40 in den Gehörgang 31 eines Ohrs 30 (in Fig. 5 angedeutet) eingeführt. Das Verfahren wird nachfolgend unter Bezugnahme auf die Figuren 7 und 8 erläutert.

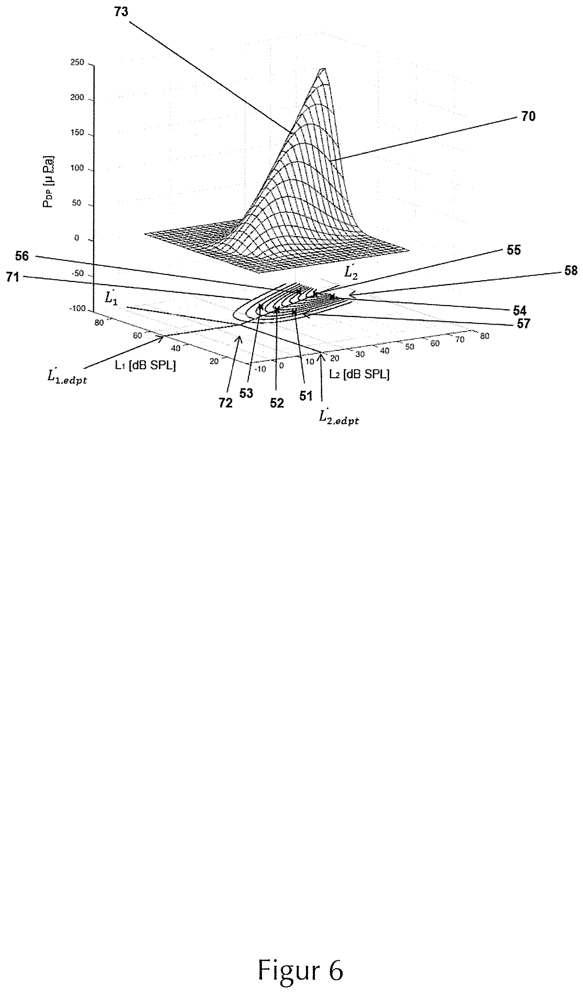

Zunächst ist aber in Figur 6 beispielhaft eine Modellfunktion dargestellt, deren dreidimensionaler Graph 70 einer Modellpegelkarte entspricht. Die beispielhaft dargestellte Modellfunktion beruht auf den Messdaten von $p \geq 2$, vorliegend $p = 6$, normalhörenden Individuen mit $N \geq 40$, vorliegend $N = 47$, gemessenen unterschiedlichen Pegelpaaren $\{L_1^{(1\ldots N)}, L_2^{(1\ldots N)}\}$ bei Anregungsfrequenzen $f_2 = 2$ kHz und $f_1 = 1{,}67$ kHz. Die Anregungsfrequenzen $f_2$ und $f_1$ eines Pegelpaares $\{L_1, L_2\}$ sind dabei vorzugsweise über ein Frequenzverhältnis $f_2/f_1 = 1{,}2$ verknüpft. Dabei wurde ein bestimmtes Distorsionsprodukt ausgewertet, welches bevorzugt bei der Frequenz $f_{dp} = 2f_1 - f_2$ liegt. Dabei bedeuten die hochgestellten Indizes in den Klammern den 1. bis N. Messpunkt.

Die Modellfunktion definiert einen näherungsweise linear ansteigenden Grat 73, dem näherungsweise linear miteinander verknüpfte $\{L_1^{(G)}, L_2^{(G)}\}$ Pegelpaare zugeordnet sind. Linien quer zum Grat können durch die Beziehung $L_2 + aL_1 = C$ definiert werden, wobei C eine beliebige Konstante, und wobei $a$ der Steigungsparameter der Projektion des Grates auf die $\{L_1, L_2\}$-Ebene ist. Im mathematischen Sinne ist die Lage des Grates durch eine hintereinanderhängende Schar von Gradientenvektoren des Skalarfeldes, das durch die DPOAE gebildet wird, definiert, wobei alle anderen durch Gradientenvektoren gebildeten Feldlinien auf diesen Grat zulaufen und einschwenken. In die unterhalb der Modellpegelkarte eingezeichnete, der Deutlichkeit halber um $p_{dp} = 100$ µPa verschobene $\{L_1, L_2\}$-Ebene 71 ist das transformierte $\{L_1', L_2'\}$-Koordinatensystem 72 eingezeichnet, welches durch Verschiebung des Ursprungs nach $\{L_{1,edpt}, L_{2,edpt}\}$ und Drehung um arctan(a) zustande kommt, sowie Höhenlinien der Pegelkarte im 20 µPa-Abstand. Die $L_2'$-Achse entspricht der Projektion des Grates der Pegelkarte auf die $\{L_1, L_2\}$-Ebene. Die $L_1'$-Achse schneidet den der Pegelkarte angenäherten Modell-Hügel orthogonal. Dieser Schnitt durch den Hügel quer zum Grat wird durch eine Parabel 2. Ordnung angenähert, deren Spreizung durch einen Parameter c gegeben ist, und der Eingang in folgende Gleichung findet:

$$L_{dp}' = -c(L_1')^2 + L_{dp}'^{(G)}$$

mit

$$L_{dp}'^{(G)} = 20 \, log_{10}(m(L_2'))$$

$L_{dp}'$ und $L_{dp}'^{(G)}$ ist dabei der Pegel einer beliebigen bzw. einer auf dem Grat befindlichen DPOAE und m ist die Steigung des Grats entlang der $L_2'$-Achse.

Das $\{L_1', L_2'\}$ - Koordinatensystem befindet sich in der von dem bekannten Koordinatensystem $\{L_1, L_2\}$ der Primärtonpegel aufgespannten Fläche. Die vorhergehend bereits angesprochene Koordinatentransformation lässt sich z. B. wie folgt ausdrücken:

$$L_1' = (L_1 - L_{1,edpt}) \cos(\varphi) - (L_2 - L_{2,edpt})\sin(\varphi)$$

$$L_2' = (L_1 - L_{1,edpt}) \sin(\varphi) + (L_2 - L_{2,edpt})\cos(\varphi)$$

Dabei entspricht die Projektion des Grates des $L_{dp}$-Hügels auf die $\{L_1, L_2\}$-Ebene der $L_2'$-Achse. Ferner entspricht der Punkt $\{L_{2,edpt}, L_{1,edpt}\}$ dem Fußpunkt des Grates des $L_{dp}$-Hügels, und $\varphi$ ist der Winkel zwischen der $L_2$-Achse und der Projektion des Grates des $L_{dp}$-Hügels auf die $\{L_1, L_2\}$-Ebene, gegeben durch die bereits genannte $L_2'$-Achse. Der Winkel $\varphi$ ist also der Winkel, um den die $L_2'$-Achse gegenüber der $L_2$-Achse gedreht ist. Der Fußpunkt des Grates kann in weiterem Sinne als äquivalent, aber nicht identisch zum "estimated distorsion product level" (edpt) interpretiert werden, wie er aus [P. Boege and T. Janssen., J. Acoust. Soc. Am., 111(4): 1810-1818, 2002] bekannt ist.

Die Modellfunktion für die Pegelkarte kann für den Gültigkeitsbereich positiver $L_{dp}$ durch fünf freie Parameter beschrieben werden: $a$; $b$; $c$; $L_{2,edpt}'$; $m$. Um aus Messwerten diese Fläche berechnen zu können, werden also mindestens 5 DPOAE benötigt.

Das Verfahren beruht auf der Anpassung der dreidimensionalen Modellfunktion an eine grob abgetastete dreidimensionale DPOAE-Pegelkarte mit vorzugsweise wenigstens 5 Messungen. In einem ersten Ausführungsbeispiel des Verfahrens zur automatischen Bestimmung einer individuellen Funktion einer DPOAE-Pegelkarte mit $p_{dp} = f(L_1, L_2)$ eines menschlichen oder tierischen Gehörs werden dem Gehör eines Individuums von dem aus Figur 5 ersichtlichen System n vordefinierte, z.B. n = 6 vordefinierte, Anregungspegelpaare $\{L_1, L_2\}$ 51, 52, 53, 54, 55, 56 präsentiert. Diese sechs vordefinierten Anregungspegelpaare $\{L_1, L_2\}$ 51, 52, 53, 54, 55, 56 sind beispielhaft in Figur 6 in der $\{L_1, L_2\}$-Ebene markiert. Aus diesen 6 vordefinierten Anregungspegelpaaren $\{L_1, L_2\}$ 51, 52, 53, 54, 55, 56, die in zwei Subsequenzen 57, 58 präsentiert werden, werden dann DPOAE ermittelt, die über das Verfahren zur Ermittlung der individuellen Funktion einer DPOAE-Pegelkarte genutzt werden.

Gemäß Figur 7 wird in einem ersten Schritt 110 des Verfahrens zunächst die bereits beschriebene Modellfunktion aus dem nicht-flüchtigen Speicher 16 in die Rechnereinheit 10 des Systems 1, bzw. den Arbeitsspeicher 15 der Rechnereinheit 10, eingelesen. Nach dem Einlesen der Modellfunktion werden von dem System 1 im zweiten Schritt 120 eine Anzahl von unterschiedlichen Pegelpaaren $\{L_1^{(1...n)}, L_2^{(1...n)}\}$ eines Anregungsfrequenzpaars $\{f_1, f_2\}$ über die Tonausgabemittel 21, 22 der Sonden-Einheit 20 ausgegeben bzw. einem Individuum präsentiert und die korrespondierenden DPOAE des Individuums über die Tonaufnahmemittel 23 erfasst, wobei wenigstens das erste Pegelpaar $\{L_1^{(1)}, L_2^{(1)}\}$ vordefiniert ist und wobei n << N ist. Die korrespondierenden DPOAE werden mittels der AD/DA-Wandlereinheit 12 der Rechnereinheit 10 zur Weiterverarbeitung zugeleitet. Dabei bedeuten die hochgestellten Indizes in den Klammern den 1. bis n. Messpunkt.

Der zweite Schritt 120 beinhaltet in einer ersten Variante des Verfahrens, das als adaptive Variante bezeichnet werden kann, eine Reihe von Subschritten 121 bis 127, die nachfolgend mit Bezug auf Figur 8 näher erläutert werden.

Gemäß Figur 8 wird in einem ersten Subschritt 121 des zweiten Schritts 120 zunächst ein Startpegel $\{L_1^{(1)}, L_2^{(1)}\}$ (vorzugsweise ein Pegel $L_1$ von 67 $\pm$ 10 dB und ein Pegel $L_2$ von 57 $\pm$ 10 dB) für ein erstes Pegelpaar aus dem nicht-flüchtigen Speicher 16 in die Rechnereinheit 10 eingelesen. Ferner werden in diesem Subschritt 121 die Schrittweiten $\Delta L_1$, $\Delta L_2$ für die weiteren Pegelpaare $\{L_1^{(2...n)}, L_2^{(2...n)}\}$ sowie Schwellen für Suchrichtungsentscheidungen $L_{dp,min}^{(G,1)}$; $\text{SNR}_{min}$) in die Rechnereinheit 10 eingelesen. $\text{SNR}_{min}$ bezeichnet dabei das gewünschte SNR (signal-to-noise ratio - Rauschabstand) und $L_{dp,min}^{(G,1)}$ bezeichnet dabei den DPOAE-Pegel auf dem Grat, der mindestens entlang einer ersten Subsequenz von k Subsequenzen vorliegen muß, damit eine nächste Subsequenz unterhalb der ersten noch mit ausreichendem SNR erwartet werden kann. Wird dieser Wert nicht erreicht, dann wird die nächste Subsequenz oberhalb der ersten, also grataufwärts, abgetastet, um eine zu lange Messzeit zum Erreichen eines ausreichenden SNR zu vermeiden. Die Schrittweiten $\Delta L_1$, $\Delta L_2$ bezeichnen den Pegelabstand von zwei aufeinanderfolgenden Pegelpaaren wobei $\Delta L_1$ insbesondere einen Wert von $\Delta L_1$ = 4 bis 14 dB, vorzugsweise von 6 bis 10 dB aufweist und wobei $\Delta L_2$ = 0 bis -2,78 dB ist, vorzugsweise $\Delta L_2$ = - 1,52 bis -2,78 dB ist.

In einem zweiten Subschritt 122 des zweiten Schritts erfolgen nun die Messungen der ersten Subsequenz von k Subsequenzen quer zum vermuteten Grad der individuellen Funktion einer Pegelkarte. Die DPOAE werden dabei bei den bereits oben beschriebenen Anregungsfrequenzen $f_2$ = 2 kHz und $f_1$ = 1,67 kHz durchgeführt. Die Anregungsfrequenzen $f_2$ und $f_1$ eines Pegelpaares $\{L_1, L_2\}$ sind dabei vorzugsweise über ein Frequenzverhältnis von etwa $f_2/f_1$ = 1,2 verknüpft. Die Subsequenz gehört dabei zu einer Anzahl von $k$ Subsequenzen, wobei k $\geq 2$ und $\leq 5$ ist. In jeder Subsequenz werden dabei eine Anzahl von $n_k$ Pegelpaaren $\{L_1, L_2\}$ gemessen.

Der Startpegel $\{L_1^{(1)}, L_2^{(1)}\}$ wird entsprechend den festgelegten Schrittweiten $\Delta L_1$, $\Delta L_2$ variiert entsprechend

der Formel $L_1^{(n+1)} = L_1^{(n)} + \Delta L_1' \cos(\varphi)$ und der weiteren Formel $L_2^{(n+1)} = L_2^{(n)} - \Delta L_1' \sin(\varphi)$. Wird in die gemessene Subsequenz eine absteigende oder keine ansteigende Flanke gemessen, wird die Suchrichtung umgekehrt und $\Delta L_1' = -\Delta L_1'$

In einem dritten Subschritt 123 wird überprüft, ob wenigstens drei valide DPOAE gemessen wurden. Ist diese Überprüfung positiv, das heißt es wurden drei valide DPOAE gemessen, geht das Verfahren weiter zum nächsten Subschritt 124. Wurden keine drei validen DPOAE gemessen, dann erfolgt eine Wiederholung der Messung, bei der aus dem ursprünglichen Anregungspegel $L_1^{(alt)}$, $L_2^{(alt)}$ ein neuer Anregungspegel $L_1^{(1)}$, $L_2^{(1)}$ nach

$$L_2^{(1)} = L_2^{(alt)} + \Delta L_2' \cos(\varphi),$$

$$L_1^{(1)} = L_1^{(alt)} + \Delta L_2' \sin(\varphi)$$

bestimmt wird.

In dem nachfolgenden vierten Subschritt 124 erfolgt die Bestimmung der Lage des Grates der individuellen Funktion anhand der drei valide bestimmten Messwerte, z.B. durch Lösung einer Parabelgleichung und Auffinden des individuellen Maximums nach der Funktion:

$$L_{dp}^{\prime(G,1)} = f(L_1^{(1..3)}, L_2^{(1..3)})$$

Hier bedeutet $L_{dp}^{\prime(G,1)}$ denjenigen Punkt auf dem geschätzten Grat der individuellen Modellfunktion, dessen zugehöriges Anregungspegelpaar auf der durch $\{L_1^{(1..3)}, L_2^{(1..3)}\}$ gebildeten Linie liegen. Die Lage des Grates der individuellen Funktion bei den höheren Anregungspegeln $L^{(1..3)}$ ist nun also bereits aus dem Subschritt 124 bekannt, die Steigung des Grates, d.h. der Parameter m, jedoch nicht.

Im nachfolgenden fünften Subschritt 125 des zweiten Schritts 120 erfolgt eine Messung einer zweiten Subsequenz entlang des vermuteten Grates, wobei lediglich ein Messwert ermittelt wird. Die Messung erfolgt nach der Formel

$$L_1^{(4)} = L_{dp}^{\prime(G,1)} - \Delta L_2' \sin(\varphi)$$

Unterschreitet $L_{dp}^{\prime(G,1)}$ eine voreingestellte Grenze $(L_{dp,min}^{\prime(G,1)})$, wird dieser Schritt zu höheren Pegeln hin ausgeführt ($\Delta L_2 = -\Delta L_2$).

Der dort gemessene Wert des DPOAE $(L_{dp}^{(4)})$ wird nachfolgend im sechsten Subschritt 126 dazu verwendet, die individuelle Steigung des Grates, m, zu bestimmen.

In dem sechsten Subschritt 126 des zweiten Schritts 120 erfolgt die Berechnung der individuellen Steigung des Grates nach der Formel $m=f(L_{dp}^{(G,1)}, L_1^{(G,1)}, L_2^{(G,1)}, L_{dp}^{(4)}, L_1^{(4)}, L^{(4)})$. Anhand der ermittelten Steigung m des Grates erfolgt nun die Festlegung eines Startpegels $L_1^{(5)}$, $L_2^{(5)}$ für die dritte Subsequenz.

Im siebten Subschritt 127 des zweiten Schritts 120 erfolgen nun die Messungen der dritten Subsequenz quer zu dem vermuteten Grat der Funktion: Es erfolgt eine Variation von $\Delta L_1'$ in $L_1^{(n+1)} = L_1^{(n)} + \Delta L_1' \cos(\varphi)$.

Vorzugsweise liegen mindestens die Hälfte der Pegelpaare $\{L_1, L_2\}$, bei denen gemessen wird, um mindestens 5 dB zu beiden Seiten abseits der Gruppe der dem Grat (der Modellfunktion) zugeordneten $\{L_1^{(G)}, L_2^{(G)}\}$ Pegelpaare.

Mit den gemäß dem zweiten Schritt 120 und seinen Subschritten 121 bis 127 ermittelten Messwerten, erfolgt nun eine Anpassung der bereits vorgestellten Modellfunktion an die gewonnenen Messwerte in einem dritten Schritt 130 in der Rechnereinheit 10. Hierbei wird an die gemessenen DPOAE-Werte die dreidimensionale Modellfunktion $p_{dp,M} = f(L_1, L_2)$ angepaßt. Die Anpassung erfolgt mit den mathematischen Methoden der Ausgleichsrechnung, z.B. mit der Methode der kleinsten Quadrate, d.h., mit der iterativen Minimierung der Differenz zwischen den $n$ Messwerten und den Werten der Modellfunktion $p_{dp,M} = f(L_1, L_2)$ an die gemessenen $n$ DPOAE (enstprechend den zugehörigen $L_1$, $L_2$-Koordinaten) bis zum Erhalt einer individuellen Funktion $p_{dp,1} = f(L_1, L_2)$ mit individuellen Parametern einer DPOAE-Funktion und -Pegelkarte des Individuums durch die Rechnereinheit 10. Man erhält damit auf einfache Weise eine individuelle Funktion / Pegelkarte des Gehörs eines Individuums mit stark verringertem messtechnischem Aufwand in kurzer Zeit.

In einem vierten Schritt 140 erfolgt nun die Ausgabe der individuell angepassten Funktion und ihrer Funktionsparameter in einem Ausgabemittel 11 des Systems 1, wie z.B. einem Display, Monitor, Drucker, etc.. Die ausgegebenen Funktionsparameter beinhalten dabei insbesondere die bereits oben beschriebenen Parameter $a$; $b$; $c$; $L'_{2,edpt}$ ; und die Steigung des Grates m. Das Ausgabemittel 11 kann, wie bereits erwähnt, auch in Form einer Schnittstelle realisiert sein, über die ein externes Ausgabegerät, wie z.B. ein Drucker oder ein Monitor an das System 1 angeschlossen werden können.

In einem möglichen weiteren Verfahrensschritt, kann die ermittelte individuelle Funktion einer DPOAE-Pegelkarte und deren Parameter von der Rechnereinheit 10 in dem nicht-flüchtiger Speicher 16 abgespeichert werden. Auch können die gemessenen Rohdaten von der Rechnereinheit 10 in dem nicht-flüchtiger Speicher 16 abgespeichert werden. Die abgespeicherten Daten können von der Rechnereinheit 10 z.B. zur kontinuierlichen Erweiterung des, der Modellfunktion einer Pegelkarte zugrundeliegenden Datensatzes verwendet werden.

Aus der erhaltenen individuell angepassten Funktion und den dazugehörigen Funktionsparametern können folgende Erkenntnisse gewonnen werden:

- Es kann eine angenäherte Distorsionsproduktschwelle berechnet werden, die Auskunft über die Schwelle des Eingangssignals für die inneren Haarzellen des gemessenen Gehörs liefert. Als entsprechenden Parameter kann $L_{2,edpt}$ angesehen werden.

- Die Breite des Grates, in der Funktion durch den Parameter c beschrieben, ist ein Maß für die Kompression und damit für die Frequenzauflösung der zugrundeliegenden Wanderwellen im gemessenen Gehör.

- Die Lage und der Winkel, in der Funktion ausgedrückt durch die Parameter $a$; $b$ enthält Informationen über die Natur eines Hörverlustes: Bei einem reinen Schallleitungsverlust verändert sich der Winkel (in der Funktion ausgedrückt durch den Parameter a) nicht, stattdessen verschiebt sich der Hügel in erster Näherung in gleichem Maße in Richtung höherer $L_1$- und $L_2$-Pegel. Wenn sich z.B. die Verschiebung des Hügels (gegenüber den Normwerten, oder gegenüber einer Referenzmessung des Individuums zu einem früheren Zeitpunkt) mit der Verschlechterung der Distorsionsproduktschwelle deckt, also $\Delta L_2 \approx \Delta L_1 \approx \Delta L_{2,edpt}$ kann auf einen reinen Schallleitungsverlust geschlossen werden.

- Die Steilheit des Grates, ausgedrückt durch den Parameter m, lässt Rückschlüsse auf einen möglichen Schallleitungsverlust zu. Solange der Hörverlust unterhalb 30 dB liegt, kann erwartet werden, daß bei einem reinen Schallleitungsverlust die Steigung den Normwerten entspricht, während bei einer Abweichung gegenüber dem Normwert eine proportionale Reduzierung der retrograden Mittelohrübertragung bei $f_{dp}$ indiziert ist.

In einer alternativen Variante des Verfahrens wird anstelle der Sub-Schritte 121 bis 127 im Rahmen der Messungen des zweiten Schritts 120 eine Anzahl von $n$ festgelegten bzw. vordefinierten aber unterschiedlichen Pegelpaaren $\{L_1,L_2\}$ (wobei $n$ vorzugsweise $\geq 5$ und $\leq 12$ ist, insbesondere $\geq 5$ und $\leq 8$) vom System ausgegeben und die Reaktion des Gehörs eines Individuums auf diese Pegelpaare $\{L_1,L_2\}$ erfasst. Diese Variante kann dabei als starres Verfahren bezeichnet werden. Die Pegelpaare $\{L_1,L_2\}$ können dabei wiederum in einer Anzahl k Subsequenzen (57, 58; vgl. Fig. 6) gemessen werden, wobei $k \geq 2$ und $\leq 12$ ist. Die $n$ Pegelpaare sind dabei dann weitestgehend statisch und es erfolgt keine Anpassung der zweiten und ggf. dritten Subsequenz auf die Ergebnisse der Messungen der ersten Subsequenz, wie es bei dem vorhergehend geschilderten Verfahren der Fall ist. Die Anzahl $n$ der Pegelpaare $\{L_1, L_2\}$ ist dabei nach einer Abwägung von Meßzeit (möglichst wenig Meßpunkte) gegenüber erzielbarer Genauigkeit (möglichst viele Punkte) ausgelegt.

In diesem starren Verfahren mit fest vorgegebenen Anregungspegeln kann bspw. mit $L'_2 = 40\ \mathrm{dB}$ für die drei höheren Anregungspegel, und $L'_2 = 25\ \mathrm{dB}$ für die drei niedrigeren Anregungspegel, und innerhalb einer Gruppe von drei Anregungspegeln jeweils $L'_1 = 0 \pm 6\ \mathrm{dB}$ gemessen werden. Im $\{L_1,L_2\}$ -Koordinatensystem entspricht das den Anregungspegeln $L_2 = 68{,}1$; $65{,}6$; $63{,}1$; $42{,}8$; $45{,}3$; $40{,}3$ und $L_1 = 68{,}0$; $73{,}5$; $79{,}0$; $63{,}3$; $57{,}8$; $68{,}7$ (vgl. hierzu Fig. 6).

Dabei verfolgt die Wahl $L'_1 = 0 \pm 6\ \mathrm{dB}$ das Ziel, mit drei Punkten, die quer zur vermuteten Lage des Grates liegen, dessen Lage sicher einzumessen: bei $f_2 = 2$ kHz fällt das DPOAE mit $\Delta L'_1 \pm 6\ \mathrm{dB}$ typischerweise auf etwa 50% des Maximalwertes ab. Wenn in erster Linie normalhörende Individuen gemessen werden sollen, wie es üblicherweise bei Siebtests der Fall ist, wird die starre Anordnung gute Ergebnisse liefern. Ausreißer müssen aber erkannt werden. Dies ist über den quadratischen Fehler der Modellanpassung möglich. Ist der Fehler zu hoch, d.h. ist der rms-Fehler (rms: root-mean-square) beispielsweise größer als *5 μPa,* müssen weitere Pegelpaare gemessen werden, bis der Fehler gering genug ist. Hier bieten sich weitere $\Delta L_1'$ -Schritte an. Ebenso muß verfahren werden, wenn wegen zu geringem Rauschabstand einzelne Meßpunkte nicht registriert werden können.

Abschließend bleibt noch anzumerken, dass abweichend von dem verwendeten und vorgängig beschriebenen Frequenzverhältnis $f_2/f_1$ von 1,2 auch ein anderes Frequenzverhältnis gewählt werden kann. So kann das Frequenzverhältnis $f_2/f_1$ z. B. auch auf einen anderen geeigneten Wert zwischen 1,15 und 1,35 festgelegt werden. Ferner könnte das Frequenzverhältnis $f_2/f_1$ eine Funktion von f2 sein.

Bezugszeichenliste

1 System
2 Kabelverbindung
3 erste Leitung
4 zweite Leitung
5 dritte Leitung
6 vierte Leitung

10 Rechnereinheit

11 Ausgabemittel

12 AD/DA-Wandlereinheit

13 DA-Wandler

14 AD-Wandler

15 Arbeitsspeicher

16 nicht-flüchtiger Speicher mit gespeicherter Modellfunktion

20 Sonden-Einheit, OAE-Sonde
21 erstes Tonausgabemittel, f1-Schallgeber
22 zweites Tonausgabemittel, f2-Schallgeber
23 Tonaufnahmemittel, Mikrophon
24 Sondenspitze

30 Ohr
31 Gehörgang

40 Pfeil

51 Anregungspegelpaar $\{L_1, L_2\}$
52 Anregungspegelpaar $\{L_1, L_2\}$
53 Anregungspegelpaar $\{L_1, L_2\}$
54 Anregungspegelpaar $\{L_1, L_2\}$
55 Anregungspegelpaar $\{L_1, L_2\}$
56 Anregungspegelpaar $\{L_1, L_2\}$
57 erste Subsequenz
58 zweite/weitere Subsequenz

70 Graph / Modellpegelkarte
71 $\{L_1, L_2\}$-Ebene

72    transformiertes $\{L_1', L_2'\}$ -Koordinatensystem

73    Grat (der DPOAE Modellpegelkarte)

110    erster Verfahrensschritt

120    zweiter Verfahrensschritt

121    erster Subschritt

122    zweiter Subschritt

123    dritter Subschritt

124    vierter Subschritt

125    fünfter Subschritt

126    sechster Subschritt

127    siebter Subschritt

130    dritter Verfahrensschritt

140    vierter Verfahrensschritt

Durch das in hier beschriebene Verfahren zur automatischen Bestimmung einer individuellen Funktion einer DPOAE-Pegelkarte können Fehler bei der Extrapolation der Wachstumsfunktionen vermieden werden, die in den Verfahren zur Messung der Distorsionsproduktschwelle nach dem vorbeschriebenen Stand der Technik prinzipbedingt vorkommen können. Ferner können zusätzliche Daten erhalten werden, die dann für eine Diagnose zur Verfügung stehen. Neben der Distorsionsproduktschwelle $L_{edpt}$ und der Steigung der Wachstumsfunktion können bei dem in PCT/EP2017/000334 beschriebenen Verfahren auch Daten zur Frequenzauflösung und Kompression der zugrundeliegenden Wanderwellen und dem Schalleitungsverlust erfasst werden. Vorteilhafterweise können daher bei gleichem oder sogar geringerem Zeitaufwand für die Messpunkte vier statt im Stand der Technik zwei Informationen erhalten werden, und Schätzfehler bei den bisher erhaltenen Parametern (der Distorsionsproduktschwelle $L_{edpt}$ und der Steigung der Wachstumsfunktion) reduziert werden.

Bei der hier beschriebenen automatischen Bestimmung einer individuellen Funktion einer DPOAE-Pegelkarte werden die Primärtöne jedes Pegelpaares vorzugsweise so gewählt, dass beide Wanderwellen am $f_2$-Abbildungsort etwa gleichhohe Amplituden aufweisen. Da die $f_1$-Wanderwelle am $f_2$-Abbildungsort noch nicht ihr Maximum erreicht hat, wird sie vorzugsweise deutlich stärker angeregt, zumindest bei insgesamt moderaten Anregungspegeln, bei denen der cochleäre Verstärker aktiv ist. Liegt die Anregungspegelkombination aber deutlich neben dem individuell optimalen Pfad, kann sich aufgrund der unterschiedlichen Stärke der Wanderwellen im Bereich des $f_2$-Tons das gezeigte Phänomen abschwächen oder auch verstärken. Daher kann eine Kombination mit dem in PCT/EP2017/000334 beschriebenen Verfahren sinnvoll sein, um eine individuellen Funktion $p_{dp,l} = f(L_1, L_2)$ mit individuellen Parametern einer DPOAE-Pegelkarte zu erhalten.

Für eine genaue Beschreibung der "Pegelkartenmethode" wird auf PCT/EP2017/000334 verwiesen.

## Patentansprüche

1.    Verfahren zur Erfassung von Distorsionsprodukten otoakustischer Emissionen (DPOAE) in einem Hörorgan, umfassend die Schritte:

(a) Ausgabe eines ersten Primärtonpaares $\{f_{1,1}, L_{1,1}, f_{2,1}, L_{2,1}\}$ aus je einem ersten Primärton mit Frequenz $f_{1,1}$ und Schalldruckpegel $L_{1,1}$ und einem zweiten Primärton mit Frequenz $f_{2,1}$ und Schalldruckpegel $L_{2,1}$ mit $f_{2,1} > f_{1,1}$, und
(b) Erfassen evozierter Distorsionsprodukte otoakustischer Emissionen (DPOAE),
wobei der erste Primärton $\{f_{1,1}, L_{1,1}\}$ mit einer zeitlichen Verzögerung $t_{\text{lag}}$ nach dem zweiten Primärton $\{f_{2,1}, L_{2,1}\}$

EP 3 638 103 B1

ausgegeben wird,

wobei mindestens ein weiteres Primärtonpaar aus je einem ersten Primärton mit Frequenz $f_{1,n}$ und Schalldruckpegel $L_{1,n}$ und einem zweiten Primärton mit Frequenz $f_{2,n}$ und Schalldruckpegel $L_{2,n}$ ausgegeben wird, wobei $f_{2,n} > f_{1,n}$ ist, wobei der zweite Primärton $\{f_{2,n}, L_{2,n}\}$ des mindestens einen weiteren n-ten Primärtonpaares mit einer zeitlichen Verzögerung $t_{lag}$ nach dem ersten Primärton $\{f_{1,n}, L_{1,n}\}$ dieses Primärtonpaares ausgegeben wird, wobei $\{f_{1,1}, L_{1,1}\} = \{f_{1,n}, L_{1,n}\}$ und $\{f_{2,1}, L_{2,1}\} = \{f_{2,n}, L_{2,n}\}$ ist, und

wobei das Verfahren weiterhin einen Schritt des Vergleichs der durch Ausgabe des ersten Primärtonpaares $\{f_{1,1}, L_{1,1}, f_{2,1}, L_{2,1}\}$ evozierten DPOAE mit den durch die Ausgabe jedes n-ten weiteren Primärtonpaares $\{f_{1,n}, L_{1,n}, f_{2,n}, L_{2,n}\}$ evozierten DPOAE umfasst.

2. Verfahren nach Anspruch 1, **dadurch gekennzeichnet, dass** die Ausgabe des mindestens einen weiteren n-ten Primärtonpaares $\{f_{1,n}, L_{1,n}, f_{2,n}, L_{2n}\}$ vor oder nach der Ausgabe des ersten Primärtonpaares $(f_{1,1}, L_{1,1}, f_{2,1}, L_{2.1}\}$ erfolgt.

3. Verfahren nach einem der Ansprüche 1 oder 2, wobei n = 2 ist.

4. Verfahren nach einem der vorhergehenden Ansprüche, **dadurch gekennzeichnet, dass** erste Primärton $\{f_{1,1}, L_{1,1}\}$ und/oder $\{f_{1,n}, L_{1,n}\}$ ("$f_1$-Puls") und optional der zweite Primärton $\{f_{2,1}, L_{2,1}\}$ und/oder $\{f_{2,n}, L_{2n}\}$ ("$f_2$-Puls") gepulst präsentiert werden.

5. Verfahren nach Anspruch 4, **dadurch gekennzeichnet, dass** die Pulslänge des $f_1$-Pulses des ersten Primär-tonpaares $\{f_{1,1}, L_{1,1}\}$ kürzer ist als die Pulslänge des $f_2$-Pulses des ersten Primärtonpaares $\{f_{2,1}, L_{2,1}\}$, und/oder die Pulslänge des $f_2$-Pulses des n-ten weiteren Primärtonpaares $\{f_{2,n}, L_{1,n}\}$ kürzer ist als die Pulslänge des $f_1$-Pulses des n-ten weiteren Primärtonpaares $\{f_{1,n}, L_{1,n}\}$.

6. Verfahren nach Anspruch 4 oder 5, **dadurch gekennzeichnet, dass** der $f_1$-Puls des ersten Primärtonpaares $\{f_{1,1}, L_{1,1}; f_{2,1}, L_{2,1}\}$ vor oder nach dem Ende des $f_2$-Pulses des ersten Primärtonpaares abgeschaltet wird.

7. Verfahren nach einem der vorhergehenden Ansprüche, **dadurch gekennzeichnet, dass** die zeitliche Verzögerung $t_{lag}$ zwischen 10 ms und 0,1 ms, vorzugsweise zwischen 5 ms und 0,5 ms, beträgt.

8. Verfahren nach einem der vorhergehenden Ansprüche, **dadurch gekennzeichnet, dass** die Dauer des $f_1$-Pulses des ersten und optional jedes weiteren n-ten Primärtonpaares und/oder die Dauer des $f_2$Pulses des ersten und optional jedes weiteren n-ten Primärtonpaares größer als die Latenz der evozierten DPOAE gewählt wird, vorzugsweise mindestens doppelt, noch bevorzugter mindestens dreimal und am bevorzugtesten mindestens fünfmal so lang.

9. Verfahren nach einem der vorhergehenden Ansprüche, **dadurch gekennzeichnet**, **dadurch gekennzeichnet, dass** die Dauer des $f_1$-Pulses $\{f_{1,1}, L_{1,1}\}$ des ersten Primärtonpaares und/oder des $f_2$-Pulses $\{f_{2,n}, L_{2,1}\}$ des n-ten weiteren Primärtonpaares 200 ms oder weniger, 100 ms oder weniger, 50 ms oder weniger, zwischen 40 ms bis 1 ms, zwischen 30 ms und 2 ms oder zwischen 25 ms und 5 ms beträgt.

10. Verfahren nach einem der vorhergehenden Ansprüche, **dadurch gekennzeichnet, dass** ein Satz aus dem ersten Primärtonpaar $(f_{1,1}, L_{1,1}; f_{2,1}, L_{2,1}\}$ und dem mindestens einen weiteren Primärtonpaar $\{f_{1,n}, L_{1,n}; f_{2,n}, L_{1,n}\}$ in einem Block ausgegeben werden, der während der Messdauer mehrfach wiederholt wird.

11. Verfahren nach einem der vorhergehenden Ansprüche, **dadurch gekennzeichnet, dass** die Dauer des ersten und zweiten Primärtons jedes Primärtonpaares zwischen 2 ms bis 20 ms beträgt.

12. Verfahren nach einem der Ansprüche 10 bis 11, **dadurch gekennzeichnet, dass** in einem Block der Beginn eines Primärtonpaares mit einem zeitlichen Abstand $T_a$ auf den Beginn des im Block unmittelbar vorhergehenden Primärtonpaares folgt, wobei $T_a$ vorzugsweise > 10 ms ist.

13. Verfahren nach einem der Ansprüche 10 bis 12, **dadurch gekennzeichnet, dass** in einem Block die zweiten Anregungsfrequenzen $f_2$ von zwei unmittelbar aufeinander folgenden Primärtonpaaren mindestens eine Oktave auseinanderliegen.

14. Verfahren nach einem der Ansprüche 10 bis 13, **dadurch gekennzeichnet, dass** während der Messdauer die gemessenen Schalldruckpegel der DPOAE für Primärtonpaare gleicher zweiter Anregungsfrequenzen $f_2$ gemittelt werden.

15. Verfahren nach einem der Ansprüche 10 bis 14, **dadurch gekennzeichnet, dass** der oder jeder Block von Primärtonpaaren während einer Blockzeit präsentiert wird, die so gewählt ist, dass zwischen dem Beginn eines ersten und eines folgenden Primärtonpaares mit derselben Anregungsfrequenz $f_2$ ein zeitlicher Abstand von 30 ms bis 100 ms, vorzugsweise von zumindest 70 ms, liegt.

16. Verfahren nach einem der Ansprüche 1 bis 15, **dadurch gekennzeichnet, dass** zu Beginn der Messungen überprüft wird, ob die Frequenz $f_{dp}$ einer der DPOAE mit einer spontanen Emission (SOAE) interferiert.

17. Verfahren nach einem der Ansprüche 10 bis 16, **dadurch gekennzeichnet, dass** zumindest zwei Sätze mit zumindest teilweise hinsichtlich der zweiten Anregungsfrequenz $f_2$ unterschiedlichen Primärtonpaaren ausgewählt werden, wobei die Blöcke der einzelnen Sätze zeitlich nacheinander präsentiert werden, vorzugsweise **dadurch gekennzeichnet, dass** die Schalldruckpegel der DPOAE für alle in dem oder jedem Satz enthaltenen zweiten Anregungsfrequenzen $f_2$ bei einem jeweils der Anregungsfrequenz $f_2$ zugeordneten zweiten Schalldruckpegel $L_2$ gemessen und gemittelt werden, und die Messungen zumindest einmal für neue Schalldruckpegel $L_2$ durchgeführt werden.

18. Verfahren nach einem der vorhergehenden Ansprüche, außerdem umfassend die automatischen Bestimmung einer individuellen Funktion einer DPOAE-Pegelkarte mit $p_{dp,I} = f(L_1, L_2)$, bei der DPOAE als Schalldruck $p$ in Abhängigkeit von den Pegeln $L_1$ und $L_2$ der zur Erzeugung der DPOAE verwendeten zwei Primärtöne dargestellt werden, zur Ermittlung des optimalen DPOAE-Anregungspegels:

    - Einlesen (110) einer Modellfunktion $p_{dp,M} = f(L_1, L_2)$ mit Modellparametern einer DPOAE-Pegelkarte (70), basierend auf einer Anzahl von $N$ DPOAE-Messungen eines Anregungsfrequenzpaars $f_1$, $f_2$ mit jeweils unterschiedlichen Pegelpaaren $\{L_1^{(1...N)}, L_2^{(1...N)}\}$ bei einer Population (p) von Normalhörenden, in einen Arbeitsspeicher einer Rechnereinheit, wobei $N \geq 40$ und $p \geq 2$ ist,

    - automatische Präsentation (120) von n unterschiedlichen Pegelpaaren $\{L_1^{(1...n)}, L_2^{(1...n)}\}$ (51, 52, 53, 54, 55, 56) eines Anregungsfrequenzpaars $f_1$, $f_2$ über Tonausgabemittel (21, 22) an ein Individuum und Erfassen der korrespondierenden DPOAE des Individuums über Tonaufnahmemittel (23), wobei wenigstens das erste Pegelpaar $\{L_1^{(1)}, L_2^{(1)}\}$ (51) vordefiniert ist und wobei $n \ll N$ ist,

    - iteratives Anpassen der Modellfunktion $p_{dp,M} = f(L_1, L_2)$ an die gemessenen n DPOAE bis zum Erhalt einer individuellen Funktion $p_{dp,I} = f(L_1, L_2)$ mit individuellen Parametern einer DPOAE-Pegelkarte des Individuums durch die Rechnereinheit, und

    - Ausgabe der individuellen Funktion $p_{dp,I} = f(L_1, L_2)$ und/oder deren individueller Parameter an einem Ausgabemittel der Rechnereinheit.

**Claims**

1. A method for detecting distortion products of otoacoustic emissions (DPOAE) in an auditory organ, comprising the steps of:

    (a) output of a first primary tone pair $\{f_{1,1}, L_{1,1}, f_{2,1}, L_{2,1}\}$ each consisting of a first primary tone having a frequency $\{f_{1,1}$ and a sound pressure level $L_{1,1}$ and a second primary tone having a frequency $f_{2,1}$ and a sound pressure level $L_{2,1}$, with $f_{2,1} > f_{1,1}$, and
    (b) detection of evoked distortion products of otoacoustic emissions (DPOAE),
    wherein the first primary tone $\{f_{1,1}, L_{1,1}\}$ is output with a time delay $t_{lag}$ after the second primary tone $\{f_{2,1}, L_{2,1}\}$,
    wherein at least one further primary tone pair is output each consisting of a first primary tone having a frequency $f_{1,n}$ and a sound pressure level $L_{1,n}$ and a second primary tone having a frequency $f_{2,n}$ and a sound pressure level $L_{2,n}$, with $f_{2,n} > f_{1,n}$, wherein the second primary tone $\{f_{2,n}, L_{2,n}\}$ of the at least one further $n^{th}$ primary tone pair is output with a time delay $t_{lag}$ after the first primary tone $\{f_{1,n}, L_{1,n}\}$ of this primary tone pair,
    wherein $\{f_{1,1}, L_{1,1}\} = \{f_{1,n}, L_{1,n}\}$ and $\{f_{2,1}, L_{2,1}\} = \{f_{2,n}, L_{2,n}\}$, and

wherein the method further comprises a step of comparing the DPOAEs evoked by the output of the first primary tone pair $\{f_{1,1}, L_{1,1}, f_{2,1}, L_{2,1}\}$ with the DPOAEs evoked by the output of each $n^{th}$ further primary tone pair $\{f_{1,n}, L_{1,n}, f_{2,n}, L_{2,n}\}$.

2. The method according to claim 1, **characterised in that** the output of the at least one further $n^{th}$ primary tone pair $\{f_{1,n}, L_{1,n}, f_{2,n}, L_{2,n}\}$ occurs before or after the output of the first primary tone pair $\{f_{1,1}, L_{1,1}, f_{2,1}, L_{2,1}\}$.

3. The method according to any one of claims 1 or 2, wherein n = 2.

4. The method according to any one of the preceding claims, **characterised in that** the first primary tone $\{f_{1,1}, L_{1,1}\}$ and/or $\{f_{1,n}, L_{1,n}\}$ ("$f_1$ pulse") and optionally the second primary tone $\{f_{2,1}, L_{2,1}\}$ and/or $\{f_{2,n}, L_{2,n}\}$ ("$f_2$ pulse") are presented as a pulsed tone.

5. The method according to claim 4, **characterised in that** the pulse length of the $f_1$ pulse of the first primary tone pair $[f_{1,1}, L_{1,1}]$ is shorter than the pulse length of the $f_2$ pulse of the first primary tone pair $\{f_{2,1}, L_{2,1}\}$, and/or the pulse length of the $f_2$ pulse of the $n^{th}$ further primary tone pair $\{f_{2,n}, L_{2,n}\}$ is shorter than the pulse length of the $f_1$ pulse of the $n^{th}$ further primary tone pair $\{f_{1,n}, L_{1,n}\}$.

6. The method according to claim 4 or 5, **characterised in that** the $f_1$ pulse of the first primary tone pair $\{f_{1,1}, L_{1,1}; f_{2,1}, L_{2,1}\}$ is switched off before or after the end of the $f_2$ pulse of the first primary tone pair.

7. The method according to any one of the preceding claims, **characterised in that** the time delay $t_{lag}$ is between 10 ms and 0.1 ms, preferably between 5 ms and 0.5 ms.

8. The method according to any one of the preceding claims, **characterised in that** the duration of the $f_1$ pulse of the first and optionally of each further $n^{th}$ primary tone pair and/or the duration of the $f_2$ pulse of the first and optionally of each further $n^{th}$ primary tone pair is chosen to be longer than the latency of the evoked DPOAE, preferably at least twice, more preferably at least three times and most preferably at least five times as long.

9. The method according to any one of the preceding claims, **characterised in that** the duration of the $f_1$ pulse $\{f_{1,1}, L_{1,1}\}$ of the first primary tone pair and/or of the $f_2$ pulse $\{f_{2,n}, L_{2,n}\}$ of the $n^{th}$ further primary tone pair is 200 ms or less, 100 ms or less, 50 ms or less, between 40 ms and 1 ms, between 30 ms and 2 ms or between 25 ms and 5 ms.

10. The method according to any one of the preceding claims, **characterised in that** a set of the first primary tone pair $\{f_{1,1}, L_{1,1}; f_{2,1}, L_{2,1}\}$ and the at least one further primary tone pair $\{f_{1,n}, L_{1,n}; f_{2,n}, L_{2,n}\}$ are output in a block which is repeated several times during the measurement period.

11. The method according to any one of the preceding claims, **characterised in that** the duration of the first and second primary tone of each pair of primary tones is between 2 ms and 20 ms.

12. The method according to any one of claims 10 to 11, **characterised in that** in a block the beginning of a primary tone pair follows the beginning of the primary tone pair immediately preceding in the block with a time interval $T_a$, where $T_a$ is preferably > 10 ms.

13. The method according to any one of claims 10 to 12, **characterised in that** in a block the second excitation frequencies $f_2$ of two immediately successive primary tone pairs are at least one octave apart.

14. The method according to any one of claims 10 to 13, **characterised in that** during the measurement period, for primary tone pairs of equal second excitation frequencies $f_2$, the measured sound pressure levels of the DPOAE are averaged.

15. The method according to any one of claims 10 to 14, **characterised in that** the or each block of primary tone pairs is presented during a block time which is selected such that there is a time interval of 30 ms to 100 ms, preferably of at least 70 ms, between the beginning of a first and a subsequent primary tone pair having the same excitation frequency $f_2$.

16. The method according to any one of claims 1 to 15, **characterised in that** at the beginning of the measurements it is checked whether the frequency $f_{dp}$ of one of the DPOAEs interferes with a spontaneous emission (SOAE).

**17.** The method according to any one of claims 10 to 16, **characterised in that** at least two sets with at least partially different primary tone pairs with respect to the second excitation frequency $f_2$ are selected, wherein the blocks of the individual sets are presented one after the other in time, preferably **characterised in that** that the sound pressure levels of the DPOAEs are measured and averaged for all second excitation frequencies $f_2$ contained in the or each set at a second sound pressure level $L_2$ respectively assigned to the excitation frequency $f_2$, and the measurements are carried out at least once for new sound pressure levels $L_2$.

**18.** The method according to any one of the preceding claims, further comprising automatically determining an individual function of a DPOAE level map with $p_{dp,I} = f(L_1, L_2)$, wherein DPOAE is represented as sound pressure $p$ as a function of the levels $L_1$ and $L_2$ of the two primary tones used to generate the DPOAE, to determine the optimal DPOAE excitation level:

- reading (110) a model function $p_{dp,M} = f(L_1, L_2)$ with model parameters of a DPOAE level map (70), based on a number of $N$ DPOAE measurements of an excitation frequency pair $f_1, f_2$ with respectively different level pairs $\left\{L_1^{(1 \dots N)}, L_2^{(1 \dots N)}\right\}$ in a population (p) of normal-hearing persons, into a working memory of a computer unit, where $N \geq 40$ and $p \geq 2$,

- automatic presentation (120) of n different level pairs $\left\{L_1^{(1 \dots n)}, L_2^{(1 \dots n)}\right\}$ (51, 52, 53, 54, 55, 56) of an excitation frequency pair $f_1, f_2$ via sound output means (21, 22) to an individual and detection of the corresponding DPOAE of the individual via sound recording means (23), wherein at least the first level pair $\left\{L_1^{(1)}, L_2^{(1)}\right\}$ (51) is predefined and wherein $n \ll N$,

- iterative adaptation of the model function $p_{dp,M} = f(L_1, L_2)$ to the measured n DPOAEs until an individual function $p_{dp,I} = f(L_1, L_2)$ with individual parameters of a DPOAE level map of the individual is obtained by the computer unit, and

- output of the individual function $p_{dp,I} = f(L_1, L_2)$ and/or its individual parameters to an output means of the computer unit.

## Revendications

**1.** Procédé pour la détection de produits de distorsion d'émissions oto-acoustiques (DPOAE) dans un organe auditif, comportant les étapes suivantes :

(a) émission en sortie d'une première paire de tons primaires {$f_{1,1}$, $L_{1,1}$, $f_{2,1}$, $L_{2,1}$} à partir respectivement d'un premier ton primaire ayant une fréquence $f_{1,1}$ et un niveau de pression sonore $L_{1,1}$ et d'un deuxième ton primaire ayant une fréquence $f_{2,1}$ et un niveau de pression sonore $L_{2,1}$ avec $f_{2,1} \geq f_{1,1}$, et

(b) détection de produits de distorsion d'émissions oto-acoustiques (DPOAE) évoqués, dans lequel le premier ton primaire {$f_{1,1}$, $L_{1,1}$} est émis en sortie avec un décalage temporel $t_{\text{lag}}$ après le deuxième ton primaire {$f_{2,1}$, $L_{2,1}$}, dans lequel au moins une autre paire de tons primaires comprenant respectivement un premier ton primaire ayant une fréquence $f_{1,n}$ et un niveau de pression sonore $L_{1,n}$ et un deuxième ton primaire ayant une fréquence $f_{2,n}$ et un niveau de pression sonore $L_{2,n}$ est émise en sortie, dans lequel $f_{2,n} > f_{1,n}$, dans lequel le deuxième ton primaire {$f_{2,n}$, $L_{2,n}$} de l'au moins une autre n-ième paire de tons primaires est émis en sortie avec un décalage temporel $t_{\text{lag}}$ après le premier ton primaire {$f_{1,n}$, $L_{1,n}$} de cette paire de tons primaires, dans lequel {$f_{1,1}$, $L_{1,1}$} = {$f_{1,n}$, $L_{1,n}$} et {$f_{2,1}$, $L_{2,1}$} = {$f_{2,n}$, $L_{2,n}$}, et le procédé comportant en outre une étape de la comparaison des DPOAE évoqués par émission en sortie de la première paire de tons primaires {$f_{1,1}$, $L_{1,1}$, $f_{2,1}$, $L_{2,1}$} aux DPOAE évoqués par l'émission en sortie de chaque n-ième autre paire de tons primaires {$f_{1,n}$, $L_{1,n}$, $f_{2,n}$, $L_{2,n}$}.

**2.** Procédé selon la revendication 1, **caractérisé en ce que** l'émission en sortie de l'au moins une autre n-ième paire de tons primaires {$f_{1,n}$, $L_{1,n}$, $f_{2,n}$, $L_{2,n}$} s'effectue avant ou après l'émission en sortie de la première paire de tons primaires {$f_{1,1}$, $L_{1,1}$, $f_{2,1}$, $L_{2,1}$}.

**3.** Procédé selon l'une des revendications 1 ou 2, dans lequel n = 2.

**4.** Procédé selon l'une des revendications précédentes, **caractérisé en ce que** le premier ton primaire {$f_{1,1}$, $L_{1,1}$} et/ou

$\{f_{1,n}, L_{1,n}\}$ (« impulsion $f_1$ ») et facultativement le deuxième ton primaire $\{f_{2,1}, L_{2,1}\}$ et/ou $\{f_{2,n}, L_{2,n}\}$ (« impulsion $f_2$ ») sont présentés de manière pulsée.

5. Procédé selon la revendication 4, **caractérisé en ce que** la longueur d'impulsion de l'impulsion $f_1$ de la première paire de tons primaires $\{f_{1,1}, L_{1,1}\}$ est plus courte que la longueur d'impulsion de l'impulsion $f_2$ de la première paire de tons primaires $\{f_{2,1}, L_{2,1}\}$, et/ou la longueur d'impulsion de l'impulsion $f_2$ de la n-ième autre paire de tons primaires $\{f_{2,n}, L_{2,n}\}$ est plus courte que la longueur d'impulsion de l'impulsion $f_1$ de la n-ième autre paire de tons primaires $\{f_{1,n}, L_{1,n}\}$.

6. Procédé selon la revendication 4 ou la revendication 5, **caractérisé en ce que** l'impulsion $f_1$ de la première paire de tons primaires $\{f_{1,1}, L_{1,1} ; f_{2,1}, L_{2,1}\}$ est interrompue avant ou après la fin de l'impulsion $f_2$ de la première paire de tons primaires.

7. Procédé selon l'une des revendications précédentes, **caractérisé en ce que** le décalage temporel $t_{\text{lag}}$ est compris entre 10 ms et 0,1 ms, de préférence entre 5 ms et 0,5 ms.

8. Procédé selon l'une des revendications précédentes, **caractérisé en ce que** la durée de l'impulsion $f_1$ de la première et facultativement de chaque autre n-ième paire de tons primaires et/ou la durée de l'impulsion $f_2$ de la première et facultativement de chaque autre n-ième paire de tons primaires sont choisies supérieures à la latence des DPOAE évoqués, de préférence au moins deux fois, de préférence encore au moins trois fois et idéalement au moins cinq fois plus longues.

9. Procédé selon l'une des revendications précédentes, **caractérisé en ce que** la durée de l'impulsion $f_1$ $\{f_{1,1}, L_{1,1}\}$ de la première paire de tons primaires et/ou de l'impulsion $f_2$ $\{f_{2,n}, L_{2,n}\}$ de la n-ième autre paire de tons primaires est de 200 ms ou moins, de 100 ms ou moins, de 50 ms ou moins, comprise entre 40 ms et 1 ms, entre 30 ms et 2 ms ou entre 25 ms et 5 ms.

10. Procédé selon l'une des revendications précédentes, **caractérisé en ce qu'**un ensemble de la première paire de tons primaires $\{f_{1,1}, L_{1,1} ; f_{2,1}, L_{2,1}\}$ et de l'au moins une autre paire de tons primaires $\{f_{1,n}, L_{1,n} ; f_{2,n}, L_{2,n}\}$ est émis en sortie en un bloc, lequel est répété plusieurs fois pendant la durée de mesure.

11. Procédé selon l'une des revendications précédentes, **caractérisé en ce que** la durée des premier et deuxième tons primaires de chaque paire de tons primaires est comprise entre 2 ms et 20 ms.

12. Procédé selon l'une des revendications 10 à 11, **caractérisé en ce que**, dans un bloc, le début d'une paire de tons primaires fait suite au début de la paire de tons primaires immédiatement précédente dans le bloc avec un intervalle temporel $T_a$, dans lequel $T_a$ est de préférence > 10 ms.

13. Procédé selon l'une des revendications 10 à 12, **caractérisé en ce que**, dans un bloc, les deuxièmes fréquences d'excitation $f_2$ de deux paires de tons primaires immédiatement consécutives sont espacées d'au moins une octave.

14. Procédé selon l'une des revendications 10 à 13, **caractérisé en ce que**, pendant la durée de mesure, les niveaux de pression sonore mesurés des DPOAE sont moyennés pour des paires de tons primaires de mêmes deuxièmes fréquences d'excitation $f_2$.

15. Procédé selon l'une des revendications 10 à 14, **caractérisé en ce que** le ou chaque bloc de paires de tons primaires est présenté pendant un temps de bloc, lequel est choisi de telle sorte qu'entre le début d'une première paire de tons primaires et le début d'une paire de tons primaires suivante ayant la même fréquence d'excitation $f_2$, il existe un intervalle temporel allant de 30 ms à 100 ms, de préférence d'au moins 70 ms.

16. Procédé selon l'une des revendications 1 à 15, **caractérisé en ce que**, au début des mesures, il est vérifié si la fréquence $f_{\text{dp}}$ de l'un des DPOAE interfère avec une émission spontanée (SOAE).

17. Procédé selon l'une des revendications 10 à 16, **caractérisé en ce qu'**au moins deux ensembles avec des paires de tons primaires différentes au moins en partie en ce qui concerne la deuxième fréquence d'excitation $f_2$ sont sélectionnés, dans lequel les blocs des ensembles individuels sont présentés temporellement les uns après les autres, de préférence **caractérisé en ce que** les niveaux de pression sonore des DPOAE sont mesurés et moyennés pour toutes les deuxièmes fréquences d'excitation $f_2$ contenues dans le ou chaque ensemble à un deuxième niveau de pression sonore $L_2$ associé respectivement à la fréquence d'excitation $f_2$, et les mesures sont réalisées au moins une fois pour de nouveaux niveaux de pression sonore $L_2$.

18. Procédé selon l'une des revendications précédentes, comportant en outre la détermination automatique d'une fonction individuelle d'une carte de niveaux de DPOAE avec $p_{dp,I} = f(L_1, L_2)$, dans laquelle des DPOAE sont représentés comme la pression sonore $p$ en fonction des niveaux $L_1$ et $L_2$ des deux tons primaires utilisés pour la production des DPOAE, pour l'identification du niveau d'excitation de DPOAE optimal :

- lecture (110) d'une fonction de modèle $p_{dp,M} = f(L_1, L_2)$ avec des paramètres de modèle d'une carte de niveaux de DPOAE (70), sur la base d'une pluralité de $N$ mesures de DPOAE d'une paire de fréquences d'excitation $f_1$, $f_2$ avec respectivement des paires de niveaux différentes $\{L_1^{(1...N)}, L_2^{(1...N)}\}$ dans une population (p) de personnes normo-entendantes, dans une mémoire de travail d'une unité de calcul, dans lequel $N \geq 40$ et $p \geq 2$,
- présentation automatique (120) de n paires de niveaux différentes $\{L_1^{(1...n)}, L_2^{(1...n)}\}$ (51, 52, 53, 54, 55, 56) d'une paire de fréquences d'excitation $f_1$, $f_2$ par le biais de moyens d'émission en sortie de tons (21, 22) à un individu et détection des DPOAE correspondants de l'individu par le biais de moyens de réception de tons (23), dans lequel au moins la première paire de niveaux ($L_1^{(1)}, L_2^{(1)}\}$ (51) est prédéfinie et dans lequel $n \ll N$,
- adaptation itérative de la fonction de modèle $p_{dp,M} = f(L_1, L_2)$ aux n DPOAE mesurés jusqu'à l'obtention d'une fonction individuelle $p_{dp,I} = f(L_1, L_2)$ avec des paramètres individuels d'une carte de niveaux de DPOAE de l'individu par l'unité de calcul, et
- émission en sortie de la fonction individuelle $p_{dp,I} = f(L_1, L_2)$ et/ou de ses paramètres individuels à destination d'un moyen d'émission en sortie de l'unité de calcul.

Figur 1

Figur 2

28

Figur 3

Figur 4

Figur 5

Figur 6

Einlesen der Modellfunktion — 110

Präsentation von n Pegelpaaren und Erfassung der zugehörigen Mikrophonsignale — 120

IV

Anpassung der Modellfunktion an die Messwerte — 130

Ausgabe der individuell angepaßten Modellfunktion und ihrer Funktionsparameter — 140

Figur 7

Einlesen Startpegel: $L_1^{(1)}$, $L_2^{(1)}$, sowie Schwellen für Suchrichtungsentscheidungen ($L_{dp,min}^{(G,1)}$; $SNR_{min}$) und Schrittweiten $\Delta L_1$, $\Delta L_2$.

Messung 1. Subsequenz quer zum vermuteten Grat: Variation von $\Delta L_1$ in $L_1^{(n+1)}$ $= L_1^{(n)} + \Delta L_1 \cos(\varphi)$ und $L_2^{(n+1)} = L_2^{(n)} - \Delta L_1 \sin(\varphi)$ ; wird absteigende oder keine Flanke gemessen, wird Suchrichtung umgekehrt: $\Delta L_1 = -\Delta L_1$.

Wurden drei valide DPOAE gemessen?

nein                                                                    ja

Wiederholung mit
$L_2^{(1)} = L_2^{(alt)} + \Delta L_2 \cos(\varphi)$,
$L_1^{(1)} = L_1^{(alt)} + \Delta L_2 \sin(\varphi)$

Bestimmung der Lage des Grates anhand der drei Messwerte, z.B. durch Lösung einer Parabelgleichung und Auffinden des individuellen Maximums:
$L_{dp}^{(G,1)} = f(L_1^{(G,1)}, L_2^{(G,1)})$

Messung 2. Subsequenz entlang des vermuteten Grates (ein Messwert): $L_1^{(4)} =$ $L_1^{(G,1)} - \Delta L_2 \sin(\varphi)$ und $L_2^{(4)} = L_2^{(G,1)} - \Delta L_2 \cos(\varphi)$ ; unterschreitet $L_{dp}^{(G,1)}$ eine voreingestellte Grenze ($L_{dp,min}^{(G,1)}$), wird dieser Schritt zu höheren Pegeln hin ausgeführt ($\Delta L_2 = -\Delta L_2$).

Berechnung der individuellen Steigung des Grates,
$m = f(L_{dp}^{(G,1)}, L_1^{(G,1)}, L_2^{(G,1)}, L_{dp}^{(4)}, L_1^{(4)}, L_2^{(4)})$.
Festlegung eines optimalen Startpegels $L_1^{(5)}$, $L_2^{(5)}$ für die 3. Subsequenz

Messung 3. Subsequenz quer zum vermuteten Grat: Variation von $\Delta L_1$ in $L_1^{(n+1)}$ $= L_1^{(n)} + \Delta L_1 \cos(\varphi)$ und $L_2^{(n+1)} = L_2^{(n)} - \Delta L_1 \sin(\varphi)$.

Figur 8

Figur 9

Figur 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2053877 A1
- DE 19905743 A1
- DE 102014108663
- WO 2015192969 A1
- WO 2015102969 A1
- EP 2017000334 W
- DE 102014108663 A1

**In der Beschreibung aufgeführte Nicht-Patentliteratur**

- **DALHOFF et al.** Schall- und Geschwindigkeits-DPOAE. *HNO*, 2010, vol. 58, 543-555
- **DALHOFF et al.** Two-source interference as the major reason for auditory-threshold estimation error based on DPOAE input-output functions in normal-hearing subjects. *Hearing Research*, 2013, vol. 296, 67-82
- **D. ZELLE** ; **E. DALHOFF** ; **A. W. GUMMER**. Objektive Hördiagnostik mit DPOAE. Neue Erkenntnisse zur Generierung und klinischen Anwendung. *HNO*, 2016, vol. 64 (11), 822-830
- Latencies of Extracted Distortion-Product Otoacoustic Source Components. **D. ZELLE** ; **A. W. GUMMER** ; **E. DALHOFF**. Mechanics of Hearing: Protein to Perception. 2014, vol. 1703
- **ZELLE et al.** *AIP Conference Proceedings*, 2016, vol. 1703, 090023
- **JOHNSON et al.** Influence of primary-level and primary-frequency ratios on human distortion product otoacoustic emissions. *J. Acoust. Soc. Am.*, 2006, vol. 119, 418-428
- **SHERA** ; **GUINAN**. DPOAE level map, vgl. *J Acoust Soc Am.*, February 2007, vol. 121 (2), 1003-16
- **MARTIN et al.** *J. Acoust. Soc. Am.*, vol. 127 (5), 2955-2972
- **P. BOEGE** ; **T. JANSSEN.** *J. Acoust. Soc. Am.*, 2002, vol. 111 (4), 1810-1818